(19) Europäisches Patentamt
European Patent Office
Office européen des brevets

(11) EP 4 159 213 A1

(12) EUROPEAN PATENT APPLICATION

(43) Date of publication:
**05.04.2023 Bulletin 2023/14**

(21) Application number: **21200488.1**

(22) Date of filing: **01.10.2021**

(51) International Patent Classification (IPC):
***A61K 31/4422*** *(2006.01)* ***A61P 31/20*** *(2006.01)*

(52) Cooperative Patent Classification (CPC):
**A61K 31/4422; A61P 31/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Katholieke Universiteit Leuven
3000 Leuven (BE)**

(72) Inventor: **The designation of the inventor has not yet been filed**

(74) Representative: **Arnold & Siedsma
Bezuidenhoutseweg 57
2594 AC The Hague (NL)**

# (54) PHARMACEUTICAL COMPOSITIONS FOR USE IN PREVENTION AND/OR TREATMENT AND/OR CURE OF HEPATITIS B VIRUS

(57) The present invention relates to a compound and pharmaceutical composition according to the invention, for use in the prevention and/or treatment and/or cure of hepatitis B virus. The pharmaceutical composition according to the invention provides inhibitors of human NTCP which efficient and effective inhibit hepatitis B virus. The inhibitors of human NTCP according to the invention provide an at least partially overlapping binding site between NTCP substrates and the HBV.

EP 4 159 213 A1

## Description

FIELD OF THE INVENTION

The present invention relates to pharmaceutical compositions and medicaments suitable for use in the prevention and/or treatment and/or cure of hepatitis B virus (HBV). These compounds improve inhibition and/or transporter inhibition of hepatitis B virus. Furthermore, said compounds improve functional inhibition of sodium taurocholate co-transporting polypeptide (NTCP).

BACKGROUND OF THE INVENTION

Worldwide, approximately 250 million people suffer from chronic HBV infection. These patients have a high chance of developing hepatocellular carcinoma, emphasizing the need for adequate therapy. Current medication for chronic HBV infection includes interferon alpha and nucleoside or nucleotide analogues that inhibit the HBV polymerase. However, no treatment is able to eradicate the virus and some patients require lifelong drug therapy. NTCP has been identified as the functional receptor for the HBV. It is known that the preS1 domain of the large envelope protein is essential for viral entry into hepatocytes. Binding of the preS1 domain peptide to NTCP allows to inhibit HBV entry. This led to the development of myrcludex B, a small lipopeptide derived from the preS1 domain. Although a very potent HBV entry inhibitor, myrcludex B must be administered parenterally. The preS1 domain of HBV was also able to inhibit the transport function of NTCP. Vice versa, NTCP substrates, such as taurocholate, were also able to inhibit HBV entry. This supports the hypothesis of a partially overlapping binding site. Furthermore, small molecule NTCP inhibitors such as cyclosporin A have also been shown to inhibit HBV entry. Inhibition of NTCP using small molecules is therefore an attractive strategy to prevent HBV entry into hepatocytes and could hence complement the currently approved therapies for chronic HBV infection.

The human NTCP transporter is a transporter localized at the basolateral membrane of hepatocytes. It is primarily responsible for sodium dependent uptake of taurine and glycine conjugated bile acids, but is also able to transport steroids, hormones and several drugs. Sodium independent bile acid uptake is mediated by members of the organic anion transporting polypeptide (OATP) family, especially OATP1B1 and OATP1B3. They have a wide substrate specificity including conjugated and unconjugated bile salts. Therefore, inhibition of bile salt uptake by NTCP may be largely compensated by OATPs. In addition, patients suffering from certain mutations in the gene encoding for NTCP resulting in reduced transport capacity and elevated serum bile acids did not have serious health issues.

The present invention aims at enabling efficient and effective preventing and/or treating and/or curing of a hepatitis B virus infection. The currently available therapeutics are insufficient to prevent and/or treat and/or cure HBV. Said therapeutics have limited efficacy in treating the transition towards the progressive stage of HBV, nor can they ensure recovery from chronic HBV. Therefore, patients may suffer for example from an inflammation of the liver and/or being fatigue. We have shown molecular entities inhibiting NTCP transport function. Said molecular entities contribute to new treatment options for HBV infection, in particular, said molecular entities contribute to new treatment options for chronic HBV infection.

SUMMARY OF THE INVENTION

Accordingly, in a first aspect, the present invention a compound of formula (I), or a stereoisomer, tautomer, racemic, salt, hydrate, N-oxide form, or solvate thereof, in particular for use as a medicine, more in particular for use in the prevention and/or treatment and/or cure of hepatitis B virus infection,

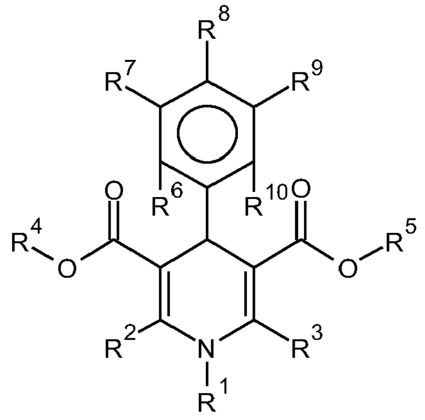

(I)

wherein:

$R^1$ is selected from the group of H, $C_1$-$C_6$ alkyl, $C_1$-$C_6$ alkene, $C_1$-$C_6$ alkyne, -$NR^{11}R^{12}$, -$OR^{13}$, $Ar^1$, $HetAr^1$,

wherein said $C_1$-$C_6$ alkyl, $C_1$-$C_6$ alkene,or $C_1$-$C_6$ alkyne is optionally substituted with one or more $Ar^3$, and wherein said $Ar^1$ or $HetAr^1$ is optionally substituted with one or more selected from the group of -$NR^{11}R^{12}$, -$OR^{13}$, wherein each occurrence of $R^{11}$, $R^{12}$ and $R^{13}$ is independently selected from the group of H, $C_1$-$C_6$ alkyl, $C_1$-$C_6$ alkene, $C_1$-$C_6$ alkyne;

$R^2$ and $R^3$ are independently selected from the group of H, $C_1$-$C_6$ alkyl, $C_1$-$C_6$ alkene, $C_1$-$C_6$ alkyne, -$NR^{14}R^{15}$ , -$OR^{16}$, wherein each occurence of $R^{14}$, $R^{15}$ and $R^{16}$ is independently selected from the group of H, $C_1$-$C_6$ alkyl, $C_1$-$C_6$ alkene, $C_1$-$C_6$ alkyne;
$R^4$ and $R^5$ are independently selected from the group of H, $C_1$-$C_6$ alkyl, $C_1$-$C_6$ alkene, $C_1$-$C_6$ alkyne, -$NR^{17}R^{18}$, $Ar^2$, $HetAr^2$, $Het^1$,

wherein said $C_1$-$C_6$ alkyl, $C_1$-$C_6$ alkene, $C_1$-$C_6$ alkyne, $Ar^2$, $HetAr^2$ and/or $Het^1$ are optionally substituted with one or more selected from the group of $C_1$-$C_6$ alkyl, $C_1$-$C_6$ alkene, $C_1$-$C_6$ alkyne, -$NR^{19}R^{20}$, -$OR^{21}$, $Ar^2$, $HetAr^2$, $Het^1$,and carbonylaldehyde; wherein each of said $C_1$-$C_6$ alkyl, $C_1$-$C_6$ alkene,and $C_1$-$C_6$ alkyne are optionally substituted with one ore more $Ar^4$;
wherein each occurence of $R^{17}$, $R^{18}$, $R^{19}$, $R^{20}$, and $R^{21}$ is independently selected from the group of H, $C_1$-$C_6$ alkyl, $C_1$-$C_6$ alkene, $C_1$-$C_6$ alkyne and carbonyl, wherein each of said $C_1$-$C_6$ alkyl, $C_1$-$C_6$ alkene,and $C_1$-$C_6$ alkyne are optionally substituted with one or more $Ar^5$;

$R^6$, $R^7$, $R^8$, $R^9$, and $R^{10}$ are independently selected from the group of H, $NO_2$, halo, CN, carbonyl

wherein $HetAr^1$ and $HetAr^2$ are each independently a 3- to 10-membered aromatic heterocycle optionally comprising 1 to 3 heteroatoms selected from O, N and S;
wherein $Ar^1$, $Ar^2$ , $Ar^3$, $Ar^4$ and $Ar^5$ are each independently a 3- to 10-membered aromatic cycle optionally comprising 1 to 3 heteroatoms selected from O, N and S; and
wherein $Het^1$ is a 3- to 10-membered heterocycle having from 1 to 3 heteroatoms selected from O, N and S.

In a particular embodiment, the present invention provides a compound for use as defined herein, wherein $R^6$, $R^7$, $R^8$, $R^9$, and $R^{10}$ are independently selected from the group of H, $NO_2$.
In a further particular embodiment, the present invention provides a compound for use as defined herein, wherein $R^7$ and/or $R^9$ are $NO_2$.
In a further particular embodiment, the present invention provides a compound for use as defined herein, wherein $R^7$ and/or $R^9$ are $NO_2$ and wherein $R^6$, $R^8$, and $R^{10}$ are H.
In a further particular embodiment, the present invention provides compound for use as defined herein, wherein the compounds of formula (I) includes the stereoisomers specifically represented by formula (Ia) or formula (lb)

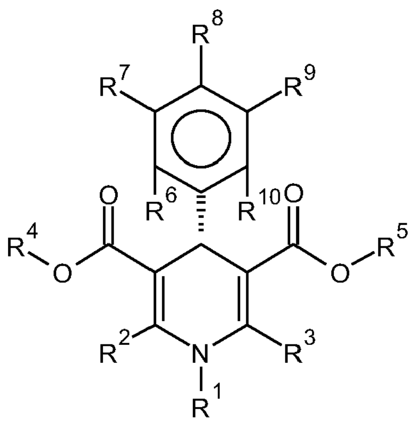

or

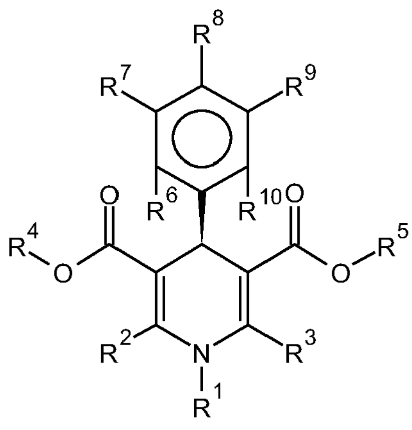

(Ia)

(Ib)

wherein $R^1$, $R^2$, $R^3$, $R^4$, $R^5$, $R^6$, $R^7$, $R^8$, $R^9$, and $R^{10}$ are defined according to different embodiments of the invention as herein disclosed.

In a further particular embodiment, the present invention provides a compound for use as defined herein, wherein $Ar^1$ and $Ar^3$ independently selected from the group of cyclopentadiene, phenyl, naphthyl, cycloheptatriene, and cyclooctatetraene; in particular $Ar^1$ and $Ar^3$ represent phenyl.

In a further particular embodiment, the present invention provides a compound for use as defined herein, wherein $Ar^2$ , $Ar^4$ and $Ar^5$ and the optionally substituted $Ar^2$ , $Ar^4$ and $Ar^5$ are each independently selected from the group of cyclopentadiene, phenyl, tolyl, xylyl, naphthyl, cycloheptatriene, cyclooctatetraene.

In a further particular embodiment, the present invention provides a compound for use as defined herein, wherein $HetAR^1$ and $HetAR^2$ are independently selected from the group of azirine, oxirene, thiirene, diazirine, azete, oxete, thiete, pyrrole, furan, thiophene, imidazole, oxathiole, oxazole, thiazole, triazole, furazan, thiadiazole, dioxazole, dithiazole, tetrazole, pyridine, pyran, thiopyran, diazine, oxazine, thiazine, dioxine, dithiin, triazine.

In a further particular embodiment, the present invention provides a compound for use as defined herein, wherein $Het^1$ is independently selected from the group of aziridine, oxirane, thiirane, diaziridine, oxaziridine, azetidine, oxetane, thietane, pyrrolidine, tetrahydrofuran, tetrahydrothiophene, imidazolidine, oxathiolidine, oxazolidine, thiazolidine, dioxolane, dithiolane, piperidine, oxane, thiane, diazinane, morpholine, thiomorpholine, dioxane, dithiane, triazinane, trioxane, trithiane.

In a further particular embodiment, the present invention provides a compound for use as defined herein, wherein $R^1$ is selected from the group of: H, $C_1$-$C_4$ alkyl,

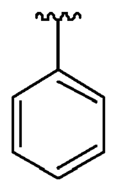

,

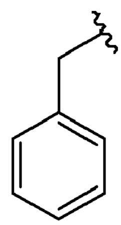

,

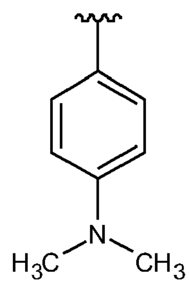

.

In a further particular embodiment, the present invention provides a compound for use as defined herein, wherein $R^2$ and $R^3$ are independently selected from the group of H, $C_1$-$C_4$ alkyl, $NH_2$.

In a further particular embodiment, the present invention provides compound for use as defined herein, wherein $R^2$ and/or $R^3$ are $-CH_3$.

In a further particular embodiment, the present invention provides a compound for use as defined herein, wherein $R^2$ and $R^3$ are the same.

In a further particular embodiment, the present invention provides a compound for use as defined herein, wherein $R^4$ and $R^5$ are independently selected from the group of: $C_1$-$C_4$ alkyl, $C_1$-$C_4$ alkene ,

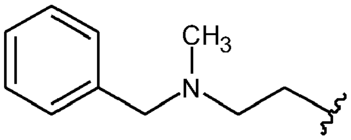 , 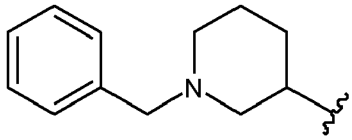

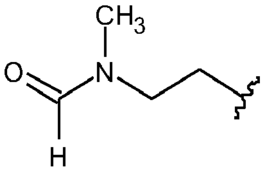 , 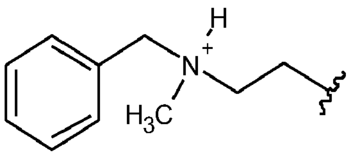 , 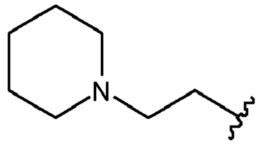

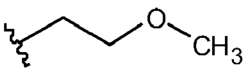 , 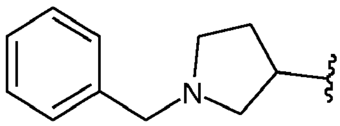 , 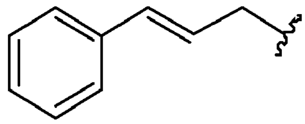

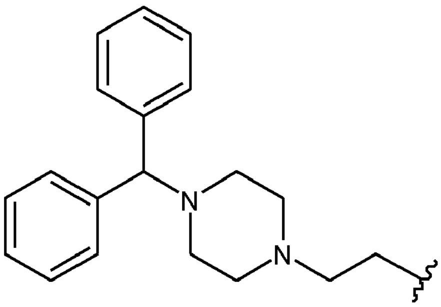 , 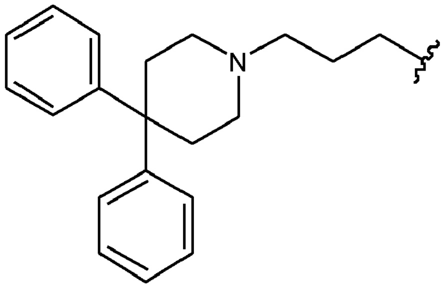 ,

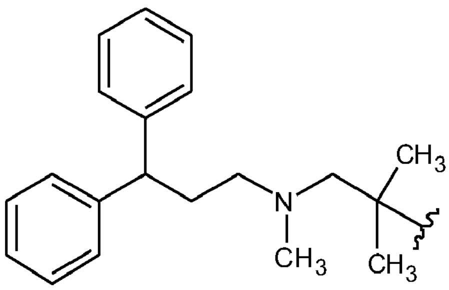 , 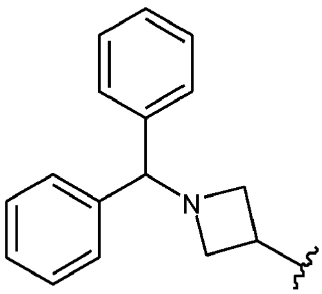

In a further particular embodiment, the present invention provides a compound for use as defined herein, wherein $R^4$ and $R^5$ are the same.

In a further particular embodiment, the present invention provides a pharmaceutical composition comprising a compound as defined herein, optionally further comprising a pharmaceutical acceptable carrier.

In a further particular embodiment, the present invention provides a pharmaceutical composition as defined herein, for use in human or veterinary medicine; in particular for use in the prevention and/or treatment and/or cure of hepatitis B virus infection.

In a further particular embodiment, the present invention provides a pharmaceutical composition as defined herein, wherein $R^6$, $R^7$, $R^8$, $R^9$, and/or $R^{10}$ are independently selected from the group of F or Cl or Br, preferebly wherein $R^6$, $R^7$, $R^8$, $R^9$, and/or $R^{10}$ are F or Cl.

DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Accordingly, as already defined herein above, the present invention provides compounds according to formula (I), or a stereoisomer, tautomer, racemic, salt, hydrate, N-oxide form, or solvate thereof, as well as pharmaceutical compositions comprising said compounds, for use in the prevention and/or treatment and/or cure of hepatitis B virus infection,

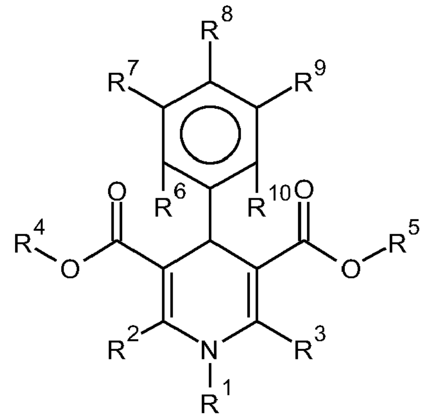

(I)

wherein:

$R^1$ is selected from the group of H, $C_1$-$C_6$ alkyl, $C_1$-$C_6$ alkene, $C_1$-$C_6$ alkyne, $-NR^{11}R^{12}$, $-OR^{13}$, $Ar^1$, $HetAr^1$,

wherein said $C_1$-$C_6$ alkyl, $C_1$-$C_6$ alkene,or $C_1$-$C_6$ alkyne is optionally substituted with one or more $Ar^3$, and wherein said $Ar^1$ or $HetAr^1$ is optionally substituted with one or more selected from the group of $-NR^{11}R^{12}$, $-OR^{13}$, wherein each occurrence of $R^{11}$, $R^{12}$ and $R^{13}$ is independently selected from the group of H, $C_1$-$C_6$ alkyl, $C_1$-$C_6$ alkene, $C_1$-$C_6$ alkyne;

$R^2$ and $R^3$ are independently selected from the group of H, $C_1$-$C_6$ alkyl, $C_1$-$C_6$ alkene, $C_1$-$C_6$ alkyne, $-NR^{14}R^{15}$ , $-OR^{16}$, wherein each occurence of $R^{14}$, $R^{15}$ and $R^{16}$ is independently selected from the group of H, $C_1$-$C_6$ alkyl, $C_1$-$C_6$ alkene, $C_1$-$C_6$ alkyne;
$R^4$ and $R^5$ are independently selected from the group of H, $C_1$-$C_6$ alkyl, $C_1$-$C_6$ alkene, $C_1$-$C_6$ alkyne, $-NR^{17}R^{18}$, $Ar^2$, $HetAr^2$, $Het^1$,
wherein said $C_1$-$C_6$ alkyl, $C_1$-$C_6$ alkene, $C_1$-$C_6$ alkyne, $Ar^2$, $HetAr^2$ and/or $Het^1$ are optinally substituted with one or more selected from the group of $C_1$-$C_6$ alkyl, $C_1$-$C_6$ alkene, $C_1$-$C_6$ alkyne, -
$NR'^{9}R^{20}$, $-OR^{21}$, $Ar^2$, $HetAr^2$, $Het^1$, and carbonylaldehyde; wherein each of said $C_1$-$C_6$ alkyl, $C_1$-$C_6$ alkene,and $C_1$-$C_6$ alkyne are optionally substituted with one ore more $Ar^4$;
wherein each occurence of $R^{17}$, $R^{18}$, $R^{19}$, $R^{20}$, and $R^{21}$ is independently selected from the group of H, $C_1$-$C_6$ alkyl, $C_1$-$C_6$ alkene, $C_1$-$C_6$ alkyne and carbonyl, wherein each of said $C_1$-$C_6$ alkyl, $C_1$-$C_6$ alkene,and $C_1$-$C_6$ alkyne are optionally substituted with one or more $Ar^5$;
$R^6$, $R^7$, $R^8$, $R^9$, and $R^{10}$ are independently selected from the group of H, $NO_2$, halo, CN, carbonyl

wherein $HetAr^1$ and $HetAr^2$ are each independently a 3- to 10-membered aromatic heterocycle optionally comprising 1 to 3 heteroatoms selected from O, N and S;
wherein $Ar^1$, $Ar^2$ , $Ar^3$, $Ar^4$ and $Ar^5$ are each independently a 3- to 10-membered aromatic cycle optionally comprising 1 to 3 heteroatoms selected from O, N and S; and wherein $Het^1$ is a 3- to 10-membered heterocycle having from 1 to 3 heteroatoms selected from O, N and S.

The compounds according to the invention provides inhibitors of human NTCP which efficient and effective inhibit HBV infection. The inhibitors of human NTCP according to the invention provide an at least partially overlapping binding site between NTCP substrates and the HBV. Therefore, the pharmaceutical composition according to the invention are potent HBV entry blockers.

Consequently pharmaceutical compositions comprising said compounds may therefore provide efficient and effective curing and/or treating and/or prevention of HBV infection. Therefore the pharmaceutical composition according to the invention may increase the health of many humans.

It was found that the compounds according to the invention comprises the ability to inhibit uptake of tauro-nor-THCA-24-DBD in NTCP-transfected cells by more than 50%.

In a preferred embodiment according to the invention, $R^6$, $R^7$, $R^8$, $R^9$, and $R^{10}$ in the compounds according to the invention are independently selected from the group of H, $NO_2$, preferably wherein $R^7$ and/or $R^9$ are $NO_2$, more preferably wherein $R^7$ and/or $R^9$ are $NO_2$ and wherein $R^6$, $R^8$, and $R^{10}$ are H.

Experiments showed that $R^7$ and/or $R^9$ being $NO_2$ provides an electron withdrawing group to formula (I). As a result such compounds according to the invention provides an even more efficient and effective response to HBV.

In a further preferred embodiment according to the invention, the compounds of formula (I) are understood to include the stereoisomers according to formula (Ia) or formula (Ib)

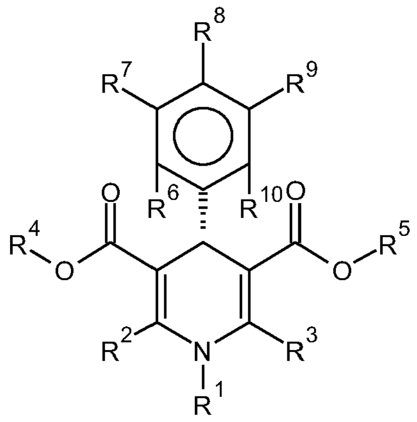

(Ia)

or

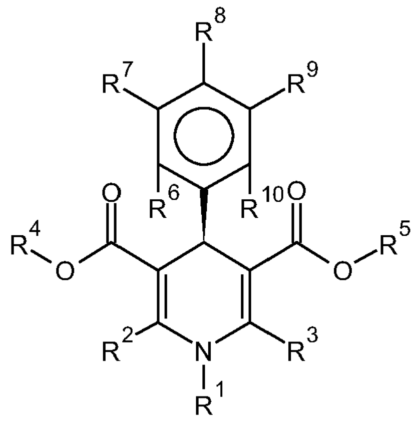

(Ib)

wherein $R^1$, $R^2$, $R^3$, $R^4$, $R^5$, $R^6$, $R^7$, $R^8$, $R^9$, and $R^{10}$ are defined according to the invention.

In yet a further preferred embodiment of the compounds according to the invention, $Ar^1$ and $Ar^3$ independently selected from the group of cyclopentadiene, phenyl, naphthyl, cycloheptatriene, and cyclooctatetraene; in particular $Ar^1$ and $Ar^3$ represent phenyl.

In yet a further preferred embodiment of the compounds according to the invention, $Ar^2$ , $Ar^4$ and $Ar^5$ and the optionally substituted $Ar^2$ , $Ar^4$ and $Ar^5$ are each independently selected from the group of cyclopentadiene, phenyl, tolyl, xylyl, naphthyl, cycloheptatriene, cyclooctatetraene.

In yet a further preferred embodiment of the compounds according to the invention, $HetAR^1$ and $HetAR^2$ are independently selected from the group of azirine, oxirene, thiirene, diazirine, azete, oxete, thiete, pyrrole, furan, thiophene, imidazole, oxathiole, oxazole, thiazole, triazole, furazan, thiadiazole, dioxazole, dithiazole, tetrazole, pyridine, pyran, thiopyran, diazine, oxazine, thiazine, dioxine, dithiin, triazine.

In yet a further preferred embodiment of the compounds according to the invention, $Het^1$ is independently selected from the group of aziridine, oxirane, thiirane, diaziridine, oxaziridine, azetidine, oxetane, thietane, pyrrolidine, tetrahydrofuran, tetrahydrothiophene, imidazolidine, oxathiolidine, oxazolidine, thiazolidine, dioxolane, dithiolane, piperidine, oxane, thiane, diazinane, morpholine, thiomorpholine, dioxane, dithiane, triazinane, trioxane, trithiane.

In yet a further preferred embodiment of the compounds according to the invention, $R^1$ is selected from the group of: H, $C_1$-$C_4$ alkyl,

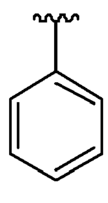

,

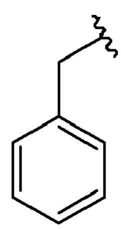

,

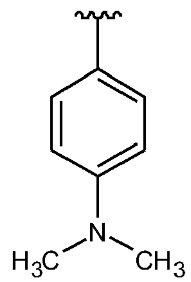

.

Experiments showed that the pharmaceutical composition comprising compounds according to the invention wherein $R^1$ is selected from the group of: H, $C_1$-$C_4$ alkyl,

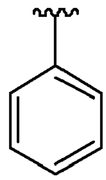 , 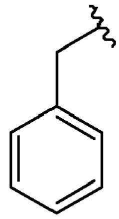 , 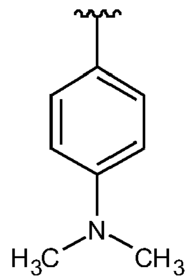 ,

provides a more efficient and effective manner to treat, cure, and/or prevent HBV.

In yet a further preferred embodiment of the compounds according to the invention, $R^2$ and $R^3$ are independently selected from the group of H, $C_1$-$C_4$ alkyl, $NH_2$. Preferably, $R^2$ and/or $R^3$ are - $CH_3$.

It is noted that $-CH_3$ refers to a methyl group.

In yet a further preferred embodiment of the compounds according to the invention, $R^2$ and $R^3$ are the same.

It was found that when $R^2$ and $R^3$ are the same, this results in a synergetic effect wherein the efficiency and effectiveness of the pharmaceutical composition according to the invention is increased.

In yet a further preferred embodiment of the compounds according to the invention, $R^4$ and $R^5$ are independently selected from the group of: $C_1$-$C_4$ alkyl, $C_1$-$C_4$ alkene ,

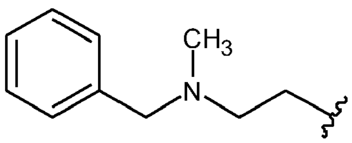 , 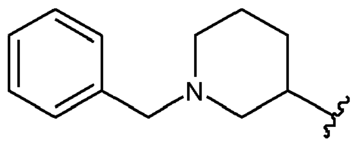

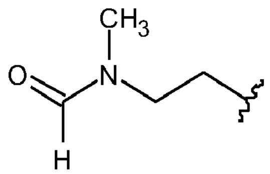 , 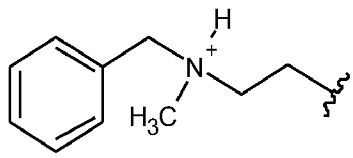 , 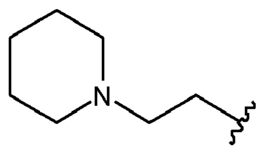

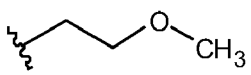 , 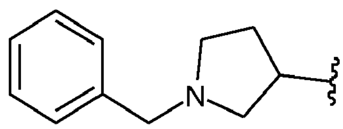 , 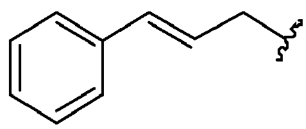

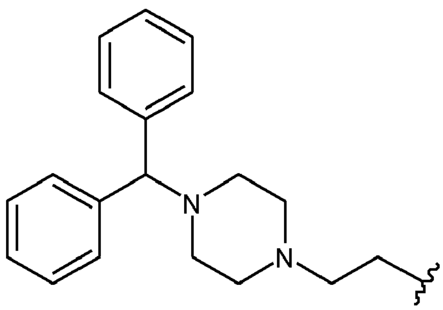 , 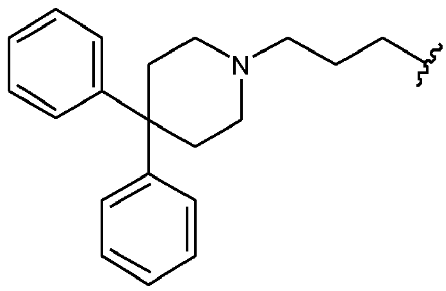 ,

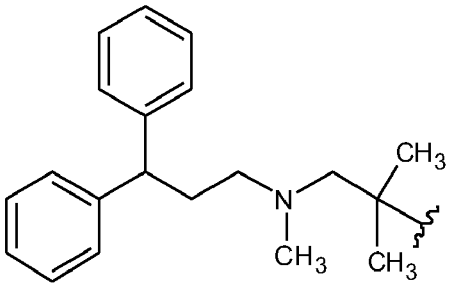 , 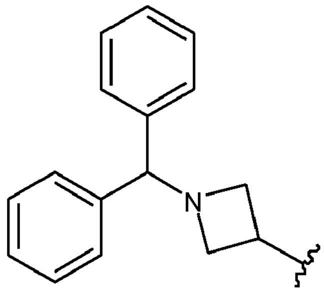

In yet a further preferred embodiment of the compounds according to the invention, $R^4$ and $R^5$ are the same.

In further embodiment the present invention provides a pharmaceutical composition comprising the compounds according to the invention. In particular a pharmaceutical composition for use as a medicine. In a further embodiment the pharmaceutical composition further comprises a pharmaceutical acceptable carrier.

In another preferred embodiment according to the invention, the pharmaceutical compostion may be used as a medicament in human or veterinary. Said pharmaceutical composition may be used in the prevention and/or treatment and/or cure of HBV infection, in particular chronic HBV infection.

It was found that the pharmaceutical compostion provided an efficient and effective prevention and/or treatment and/or cure of HBV infection.

For pharmaceutical use, the pharmaceutical compostion according to the invention may be used as a free acid or base, and/or in the form of a pharmaceutically acceptable acid-addition and/or base-addition salt (e.g. obtained with non-toxic organic or inorganic acid or base), in the form of a hydrate, solvate and/or complex, and/or in the form of a pro-drug or pre-drug, such as an ester. As used herein and unless otherwise stated, the term "solvate" includes any combination which may be formed by a pharmaceutical composition of this invention with a suitbale inorganic solvent (e.g. hydrates) or organic solvent, such as but not limited to alcohols, ketones, esters, and the like. Such asalts, hydrates, solvates, etc. and the preparation thereof will be clear to the skilled person; reference is for instance made to the slats, hydrates, solvates, etc.

The pharmaceutically acceptable salts of the pharmaceutical compostion according to the invention, i.e. in the form of water-, oil-soluble, or dispersible products, include the conventional non-toxic salts or the quarternary ammonium salts which are formed, e.g., from inorganic or organic acids or bases.

Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalene-sulfonate, nicotinate, oxalate, palmoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, *N*-methyl-*D*-glucamine, and salts with amino acids such as arginine, lysine, and so forth. In addition, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

Generally, for pharmaceutical use, the pharmaceutical composition of the inventions may be formulated as a pharmaceutical preparation comprising at least one pharmaceutically acceptable carrier, diluent or excipient, and/or adjuvant, and optionally one or more further pharmaceutically active compounds.

By means of non-limiting examples, such a formulation may be in a form suitable for oral administration, for parenteral administration (such as by intravenous, intramuscular or subcutaneous injection or intravenous infusion), for topical administration (including ocular), for administration by inhalation, by a skin patch, by an implant, by a suppository, etc.. Such suitable administration forms - which may be solid, semi-solid or liquid, depending on the manner of administration - as well as methods and carriers, diluents and excipients for use in the preparation thereof, will be clear to the skilled person.

Some preferred, but non-limiting examples of such preparations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration, which may be formulated with carriers, excipients, and diluents that are suitable per se for such formulations, such as lactose, dextrose, sucrose,

sorbitol, mannitol, starches, gum acacia, calcium phosphate, alginates, tragacanth, gelatin, calcium silicate, microcrystalline cellulose, polyvinylpyrrolidone, polyethylene glycol, cellulose, (sterile) water, methylcellulose, methyl- and propylhydroxybenzoates, talc, magnesium stearate, edible oils, vegetable oils and mineral oils or suitable mixtures thereof. The formulations can optionally contain other pharmaceutically active substances (which may or may not lead to a synergistic effect with the compounds of the invention) and other substances that are commonly used in pharmaceutical formulations, such as lubricating agents, wetting agents, emulsifying and suspending agents, dispersing agents, desintegrants, bulking agents, fillers, preserving agents, sweetening agents, flavoring agents, flow regulators, release agents, etc.. The pharmaceutical compositions may also be formulated so as to provide rapid, sustained or delayed release of the active compound(s) contained therein, for example using liposomes or hydrophilic polymeric matrices based on natural gels or synthetic polymers. In order to enhance the solubility and/or the stability of the compounds of a pharmaceutical composition according to the invention, it can be advantageous to employ α-, β- or γ-cyclodextrins or their derivatives. In particular, the present invention encompasses a pharmaceutical composition comprising an effective amount of a compound according to the invention with a pharmaceutically acceptable cyclodextrin.

In addition, co-solvents such as alcohols may improve the solubility and/or the stability of the pharmaceutical compostion. In the preparation of aqueous compositions, addition of salts of the pharmaceutical compostion of the invention can be more suitable due to their increased water solubility.

Particular reference is made to the pharmaceutical compositions, formulations (and carriers, excipients, diluents, etc. for use therein), routes of administration etc., which are known per se for analogous pyridinocarboxamides.

More in particular, the compositions may be formulated in a pharmaceutical formulation comprising a therapeutically effective amount of particles consisting of a solid dispersion of the pharmaceutical compostion or compounds of the invention and one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion is referred to as "a solid solution". Solid solutions are preferred physical systems because the components therein are usually readily bioavailable to the organisms to which they are administered.

It may further be convenient to formulate the compounds in the form of nanoparticles which have a surface modifier adsorbed on the surface thereof in an amount sufficient to maintain an effective average particle size of less than 1000 nm. Suitable surface modifiers can preferably be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the compounds according to the invention involves a pharmaceutical composition whereby the compounds are incorporated in hydrophilic polymers and applying this mixture as a coat film over many small beads, thus yielding a composition with good bio-availability which can conveniently be manufactured and which is suitable for preparing pharmaceutical dosage forms for oral administration. Materials suitable for use as cores in the beads are manifold, provided that said materials are pharmaceutically acceptable and have appropriate dimensions and firmness. Examples of such materials are polymers, inorganic substances, organic substances, and saccharides and derivatives thereof.

The preparations may be prepared in a manner known per se, which usually involves mixing at least one pharmaceutical compostion according to the invention with the one or more pharmaceutically acceptable carriers, and, if desired, in combination with other pharmaceutical active compounds, when necessary under aseptic conditions.

The pharmaceutical preparations of the invention are preferably in a unit dosage form, and may be suitably packaged, for example in a box, blister, vial, bottle, sachet, ampoule or in any other suitable single-dose or multi-dose holder or container (which may be properly labeled); optionally with one or more leaflets containing product information and/or instructions for use. Generally, such unit dosages will contain between 1 and 1000 mg, and usually between 5 and 500 mg, of the at least one compound of the invention, e.g. about 10, 25, 50, 100, 200, 300 or 400 mg per unit dosage.

The pharmaceutical compostion and/or compounds can be administered by a variety of routes including the oral, rectal, ocular, transdermal, subcutaneous, intravenous, intramuscular or intranasal routes, depending mainly on the specific preparation used and the condition to be treated or prevented, and with oral and intravenous administration usually being preferred. The at least one compound of the invention will generally be administered in an "effective amount", by which is meant any amount of a pharmaceutical compostion of the formula (I) that, upon suitable administration, is sufficient to achieve the desired therapeutic or prophylactic effect in the individual to which it is administered. Usually, depending on the condition to be prevented or treated and the route of administration, such an effective amount will usually be between 0.01 to 1000 mg per kilogram body weight day of the patient per day, more often between 0.1 and 500 mg, such as between 1 and 250 mg, for example about 5, 10, 20, 50, 100, 150, 200 or 250 mg, per kilogram body weight day of the patient per day, which may be administered as a single daily dose, divided over one or more daily doses, or essentially continuously, e.g. using a drip infusion. The amount(s) to be administered, the route of

administration and the further treatment regimen may be determined by the treating clinician, depending on factors such as the age, gender and general condition of the patient and the nature and severity of the disease/symptoms to be treated.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

For an oral administration form, the compositions of the present invention can be mixed with suitable additives, such as excipients, stabilizers, or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant.

For subcutaneous administration, the compound according to the invention, if desired with the substances customary therefore such as solubilizers, emulsifiers or further auxiliaries are brought into solution, suspension, or emulsion. The compounds of the invention can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these formulations may be prepared by mixing the compounds according to the invention with a suitable non-irritating excipient, such as cocoa butter, synthetic glyceride esters or polyethylene glycols, which are solid at ordinary temperatures, but liquefy and/or dissolve in the rectal cavity to release the drug.

In preferred embodiments, the compounds and compositions of the invention are used locally, for instance topical or in both absorbed and non-adsorbed applications.

## BRIEF DESCRIPTION OF THE DRAWINGS

Further advantages, features and details of the invention are elucidated on the basis of preferred embodiments thereof, wherein reference is made to the accompanying drawings, in which:

- Figure 1 shows the concentration-dependent uptake of tauro-nor-THCA-24-DBD in NTCP-transfected CHO cells;
- Figure 2 shows the concentration-dependent inhibition of tauro-nor-THCA-24-DBD uptake for the six known NTCP inhibitors;
- Figure 3 shows the uptake of 3 μM tauro-nor-THCA-24-DBD in NTCP-transfected CHO cells in the presence of equimolar concentrations of each compound;
- Figure 4 shows the best overlay model of the most active NTCP inhibitors (A) and superimposition of the overlay in the taurocholate binding site of NTCP (B);
- Figure 5 shows the accumulative curve of a virtual screening validation;
- Figure 6 shows the concentration-dependent inhibition of 3 μM tauro-nor-THCA-24-DBD in the presence of multiple concentrations of NTCP inhibitors; and
- Figure 7 shows the uptake of tauro-nor-THCA-24-DBD in NTCP-transfected and wild-type CHO cells.

EXAMPLES

The spectrum collection was obtained from MicroSource Discovery Stystems Inc. (Gaylordsville, CT, USA) and contains 2000 compounds with a diverse structure and origin, dissolved in 10 mM dimethylsulfoxide. An additional compound library consisting of 556 analogues was purchased from ChemDiv Inc. (San Diego, CA, USA). Dulbecco's Modified Eagle Medium (DMEM) with 1 g·$L^{-1}$ glucose, 25 mM HEPES and without L-glutamine, fetal bovine serum (FBS), L-glutamine, penicillin/streptomycin 10000 IU·$mL^{-1}$, phosphate buffered saline (PBS), Hanks' balanced salt solution (HBSS) and trypsin were obtained from Lonza SPRL (Verviers, Belgium). L-proline and Triton X-100 were obtained from Sigma-Aldrich (Schnelldorf, Germany), Geneticin sulfate from Invitrogen (Paisley, UK) and HEPES from MP Biochemical (Illkirch, France). Tauro-nor-THCA-24-DBD was purchased from GenoMembrane (Yokohama, Japan).

To culture of NTCP-transfected CHO cells, wild-type and human NTCP-transfected CHO cells were cultured from passage 20 to 45 in T-75 flasks (Greiner-Bio-One, Wemmel, Belgium) as prescribed previously (Treiber et al., 2007). The cells were placed in an incubator at 37 °C and 5% $CO_2$. The culture medium consisted of DMEM with 1 g·$L^{-1}$ glucose and 25 mM HEPES, supplemented with 10% FBS, 0.05 mg·$mL^{-1}$ L-proline, 100 IU·$mL^{-1}$ penicillin, 100 IU·$mL^{-1}$ streptomycin, 2 mM glutamine, 0.11 mg·$mL^{-1}$ sodium pyruvate. To ensure selection of NTCP-transfected cells, 0.5 mg·$mL^{-1}$ geneticin was added to the medium of the transfected cells. Cells were seeded in black 96-well plates with transparent bottom at a density of 6000 cells·$well^{-1}$ and 4000 cells·$well^{-1}$ for wild-type and transfected cells, respectively. The medium was replaced every other day and 5 mM sodium butyrate was added to induce protein expression at least 36 h before the start of the experiments. Experiments were performed on day 4 or 5 after seeding when confluence was reached.

Furthermore, to analyse the uptake experiments in NTCP-transfected CHO cells, cells were rinsed twice with 200 μL-$well^{-1}$ uptake buffer (HBSS containing 10 mM HEPES, pH 7.4) at 37 °C and subsequently preincubated with 100 μL uptake buffer for 15 minutes. For inhibition experiments, compounds were added at double concentration during preincubation. Uptake was initiated by adding 100 μL double-concentrated tauro-nor-THCA-24-DBD solution in uptake buffer. Cells were incubated for 15 minutes. The reaction was quenched by aspirating the incubation mixture and washing four times with ice cold uptake buffer. The cells were lysed with 100 μL 0.5% Triton X-100 in PBS and placed on a plate shaker (300 rpm) at room temperature for 30 minutes. Uptake of tauro-nor-THCA-24-DBD was determined using fluorescence spectroscopy (excitation 440 nm, emission 580 nm) using a Tecan Infinite 200 plate reader (Tecan Benelux, Mechelen, Belgium). Protein content of each well was determined with the BCA Protein assay (Thermo Fisher Scientific, Waltham, MA) performed according to the manufacturer's protocol. Uptake kinetics of tauro-nor-THCA-24-DBD were determined by incubating eight different concentrations in the absence of any compound. The inhibitory potential of all compounds of the Spectrum Collection (n = 2000) and the ChemDiv library (n = 556) were determined at equimolar concentrations with 3 μM tauro-nor-THCA-24-DBD. To determine the $IC_{50}$ values, at least 7 different concentrations were incubated in presence of 3 μM tauro-nor-THCA-24-DBD. All conditions were tested in triplicate.

Direct analytical interference of the ligands with the fluorescence intensity signal of the substrate was excluded. The ligands were incubated at three concentrations (5 μM, 0.5 μM and 0.05 μM) with 0.5 μM tauro-nor-THCA-24-DBD in triplicate. A one-sided Dunnett's test was performed to compare the ligands with the control, i.e. when no test compound present.

The analysis of the in vitro data for tauro-nor-THCA-24-DBD uptake in wild-type cells remained consistently below the lower limit of quantification. Signal intensities of all conditions were therefore only corrected for the blank, defined as the signal in NTCP-transfected cells in absence of substrate or any compound. Kinetic parameters of tauro-nor-THCA-24-DBD uptake were determined by fitting the Michaelis-Menten equation to the uptake values:

$$v = \frac{V_{max} \times [S]}{K_m + [S]}$$

where v is the uptake velocity, $V_{max}$ the maximum velocity, $K_m$ the substrate concentration at half maximal velocity and [*S*] the concentration of the substrate.

Uptake of tauro-nor-THCA-24-DBD in presence of compounds was expressed to uptake of the control for each batch, i.e. when no compound was present.

For determination of the $IC_{50}$, the uptake in presence of various concentrations of inhibitor was fitted to the sigmoid $E_{max}$ model:

$$E = E_{max} - (E_{max} - E_0) \times \frac{[I]^n}{[I]^n + IC_{50}^n}$$

with E the uptake of tauro-nor-THCA-24-DBD, $E_0$ the uptake at maximal inhibition by the compound, $E_{max}$ the uptake at minimal inhibition, $IC_{50}$ the concentration of the inhibitor at half maximal inhibition, *n* the Hill coefficient and [*I*] the concentration of the inhibitor. All curves were fitted with nonlinear least squares regression using R version 3.5.1.

Assuming competitive inhibition, the Cheng-Prusoff equation was used to determine the $K_i$ value:

$$K_i = \frac{IC_{50}}{1 + \frac{[S]}{K_m}}$$

where $K_i$ is the binding affinity of the inhibitor, $IC_{50}$ the concentration of the inhibitor at half maximal inhibition, [*S*] the substrate concentration, and $K_m$ the Michaelis-Menten constant of the substrate.

The Z score was used as a measure of assay quality and was expected to be ≥ 0.5 for each independent experiment:

$$Z = 1 - \frac{3\sigma_{NTCP} + 3\sigma_{WT}}{|\mu_{NTCP} - \mu_{WT}|}$$

where $\sigma_{NTCP}$ and $\sigma_{WT}$ represent the standard deviation and $\mu_{NTCP}$ and $\mu_{WT}$ the mean uptake of tauronor-THCA-24-DBD in NTCP-transfected and wild-type cells, respectively. When no individual Z score could be determined due to lacking wild-type values, the overall mean and standard deviation (SD) were used instead. Outlying values were detected with Grubb's test (Grubbs, 1950). Significance was defined as $p < 0.05$.

The data and statistical analysis comply with the recommendations on experimental design and analysis in pharmacology (Curtis et al., 2018).

Compounds with an $IC_{50}$ lower than 300 μM, including those used for validation of the *in vitro* assay, were considered NTCP inhibitors. All NTCP inhibitors and non-inhibitors were subjected to a curation process with ChemAxon's Standardizer (ChemAxon Limited, Budapest, Hungary) and included the removal of explicit hydrogen atoms, ring aromatization, normalization of specific chemotypes such as nitro groups and tautomeric forms, striping of salts and removal of small fragments. Duplicate compounds were detected with the EdiSDF tool of the ISIDA/QSPR package.

Inhibitors were clustered with ChemAxon's LibMCS (ChemAxon Limited, Budapest, Hungary) and at least nine representative compounds were selected for the external virtual screening validation set (VSVS), which also contained all non-inhibitors. The NTCP inhibitors not reserved for external validation were used for model training and selection. The most potent inhibitors ($IC_{50}$ < 6 μM) were employed for model generation (training set) and the remaining inhibitors (selection set) were used for the selection of the final model.

In addition, the sequence of human NTCP was retrieved from the UniProt database (code Q14973) (The UniProt Consortium, 2019). The corresponding three-dimensional structure of the receptor was modeled with the Swiss Model server using the structure of its apical sodium-dependent bile acid transporter (ASBT) homologue from Yersinia frederiksenii (PDB code 4n7w) as template. The binding site of taurocholate was identified from the ligand molecule cocrystalized with the Neisseria meningitidis ASBT receptor (PDB code 3zuy).

A total of 200 inhibitor conformations selected for model generation were obtained with OpenEye's Omega. Four hits for which Omega failed to generate conformations, e.g. macrocycles, were discarded from the calculations. The remaining conformations were docked into the human NTCP taurocholate binding site using OpenEye's Fred with docking resolution set to high. The top scored conformer of each compound was selected for further analyses. Compounds for which the predicted binding mode fell outside the taurocholate binding site were discarded.

The predicted binding modes of the inhibitors for which valid docking poses were predicted, were used as input to OpenEye's vROCS. Overlay hypotheses were generated using the training set, constrained to contain a maximum of five inhibitors. The three top scored overlays were saved for further investigations. The one providing the highest mean TanimotoCombo score across the selection set was selected as the best. This last filtering step ensured that the selected overlay contained features common to other NTCP inhibitors not employed for overlay generation. The best model was then used to predict the VSVS. As for the selection of the best overlay hypothesis, TanimotoCombo, that accounts for both shape and chemical overlapping, was selected as the ranking metric. Virtual screening performance was measured employing the enrichment factor (EF) for the first 0.5% (EF 0.5%), 1% (EF 1%) and 5% (EF 5%) of the screened data (Truchon and Bayly). For both model selection and virtual screening calculations, five conformers were generated for each compound with Omega.

All test compounds were used for training and validating the docking virtual screening models using the Comp-Score methodology. This algorithm exploits the information contained in the docking scoring function components to generate consensus scoring virtual screening (VS) models that outperform those based on whole scoring functions.

Primary docking to the NTCP homology model was separately performed with the Dock 6.9 (Allen et al., 2015), Gold (Jones et al., 2011) and OpenEye's Fred software (OpenEye Scientific Software, Santa Fe, New Mexico) (McGann, 2011). CompScore models were developed for each of these three primary docking alternatives. For docking with Fred, 200 conformations were generated for all NTCP inhibitors and non-inhibitors with OpenEyes's Omega (Hawkins et al., 2010). On the other hand, for docking with Dock 6.9 and Gold, one initial three-dimensional conformation per compound was obtained with Omega and AM1-BCC partial charges were added with Molcharge (OpenEye Scientific Software, Santa Fe, New Mexico).

The docking resolution in Fred was set to High. For Gold, 10 different runs were performed for each compound and search efficiency was set to 100%. The Gold's ChemPLP, Fred's Chemgauss4 and Dock's Grid scoring functions were used for primary docking. For all programs the top scored conformer of each ligand was saved. The rest of the docking software options were kept at their default values.

The binding poses predicted during each primary docking experiment were re-scored with the scoring functions according to the CompScore method (Perez-Castillo et al., 2019). The same 20 NTCP inhibitors used as training and selection sets for development of the ROCS models were used to train the CompScore models while those included in the external validation set (nine compounds) were used to challenge the predictability of the CompScore models.

Non-inhibitors were also split into training (69%) and external validation sets (31%) maintaining the same ratio of compounds in each set as for the inhibitors. To reduce the influence of dataset composition in obtaining the CompScore models, 100 different random partitions of the non-inhibitors into training and external datasets were performed. With each of these partitions, a CompScore model was trained using the datasets composed by the 20 inhibitors selected for model training and a random subset containing 69% of all non-inhibitors. CompScore models were trained to maximize the initial VS enrichment measured as Boltzmann-enhanced discrimination of receiver operating characteristic (BED-ROC) for $\alpha$ = 160.9. The scoring components present in at least 50% of the trained CompScore models were selected for the final CompScore model. The performance of this final model was evaluated over the 100 external validation sets previously generated.

The virtual screening of potential NTCP inhibitors was performed by the abovementioned developed models which were employed to screen the ChEMBL database for potential NTCP inhibitors. As for the ROCS model validation, five conformers per compound of the ChEMBL database were generated with Omega. These conformers were subsequently filtered with the selected best ligand-based ROCS model. The 0.5% top scored compounds were docked into the NTCP binding site following the procedure defined by the selected CompScore model. Finally, the 1% top scored compounds according to the CompScore model were selected as potential NTCP inhibitors.

Furthermore, in a number of examples six compounds of the top 100 potential NTCP inhibitors were selected based on availability and price: sennoside B (number 5), quercetin (number 17), fukugetin (number 24), sabutoclax (number 40), nepitrin (number 88) and baohuoside I (number 93). The $K_i$ values were determined as described earlier.

Example 1: Uptake kinetics of tauro-nor-THCA-24-DBD in NTCP-transfected CHO cells

Concentration dependent uptake of tauro-nor-THCA-24-DBD in NTCP-transfected CHO cells showed Michaelis-Menten kinetics (Figure 1). The kinetic parameters Km and Vmax were 9.2 $\pm$ 1.8 $\mu$M and 322.1 $\pm$ 18.3 pmol·mg protein$^{-1}$·min$^{-1}$, respectively. This corresponds to an uptake clearance of 35.0 $\mu$L·mg protein$^{-1}$· min$^{-1}$.

Figure 1 shows the concentration-dependent uptake of tauro-nor-THCA-24-DBD in NTCP-transfected CHO cells (n = 6). Wild-type and NTCP-transfected CHO cells were incubated for 15 minutes with eight concentrations of tauro-nor-THCA-24-DBD. Points represent mean ($\pm$ SD) of two independent experiments performed in triplicate. The line represents the best fit of the experimental data according to the Michaelis-Menten equation. The x-axis shows the concentration of tauro-nor-THCA-24-DBD in $\mu$M and the y-axis shows the tauro-nor-THCA-24-DBD uptake in pmol·mg protein$^{-1}$·min$^{-1}$.

Example 2: Inhibition of tauro-nor-THCA-24-DBD uptake by known NTCP inhibitors

To validate the assay for high throughput screening, the inhibitory potential of the six known inhibitors bosentan, cyclosporin A, rifampicin, bromosulfophthalein, ritonavir and myrcludex B was evaluated. They were able to reduce tauro-nor-THCA-24-DBD uptake in a concentration-dependent manner (Figure 2). Their respective $K_i$ values are shown in Table 1.

*Table 1: $K_i$ values of six NTCP inhibitors, which are able to inhibit uptake of tauro-nor-THCA-24-DBD in NTCP-transfected CHO cells in a concentration dependent manner*

| Compound | $K_i$ $\pm$ SE ($\mu$M) |
|---|---|
| Bosentan | 6.3 $\pm$ 1.4 |

(continued)

| Compound | $K_i$ ± SE (μM) |
|---|---|
| Cyclosporin A | 2.1 ± 0.5 |
| Rifampicin | 288.0 ± 28.0 |
| Bromosulfophthalein | 10.7 ± 3.1 |
| Ritonavir | 11.9 ± 3.5 |
| Myrcludex B | 0.052 ± 0.007 |

Figure 2 shows the concentration-dependent inhibition of tauro-nor-THCA-24-DBD uptake (15 minutes, 3 μM) for the six known NTCP inhibitors bosentan (A) and cyclosporine A (B), rifampicin (C), bromosulfophthalein (D), myrcludex B (D) and ritonavir (E). Points represent mean (± SD) of triplicate incubations. The line represents the best fit of the experimental data according to the sigmoid Emax model. The x-axis shows the concentration NTCP inhibitor in μM and the y-axis shows the tauro-nor-THCA-24-DBD uptake in pmol·mg protein$^{-1}$·min$^{-1}$.

Example 3: High throughput identification of NTCP inhibitors

The inhibitory potential of 2556 compounds was evaluated in the presence of an equimolar concentration of 3 μM tauro-nor-THCA-24-DBD (Figure 3). Forty-one compounds were able to reduce tauro-nor-THCA-24-DBD uptake for at least 50% (Table 2). The $IC_{50}$ value could be determined for 30 compounds confirming their inhibitory activity towards NTCP. The corresponding $K_i$ values ranged from 0.08 μM to 125 μM. In addition, we noticed erythromycin estolate and candesartan cilexetil among the confirmed hits. Because these two compounds are marketed prodrugs, we also determined the inhibitory potential of their active moieties erythromycin and candesartan. Only candesartan showed inhibition of tauro-nor-THCA-24-DBD uptake with a $K_i$ of 50.9 ± 9.9 μM.

*Table 2: Compounds that inhibited tauro-nor-THCA-24-DBD uptake in NTCP-transfected CHO cells for at least 50%*

| Compound | Uptake of tauro-nor-THCA-24-DBD (% of control) | $K_i$ (μM) |
|---|---|---|
| Tannic acid | 8.31 | 0.35 |
| Erythromycin estolate | 8.49 | 0.52 |
| Lobaric acid | 9.70 | 0.36 |
| Gossypol | 12.48 | 0.40 |
| Candesartan cilextil | 19.76 | 0.34 |
| Robustic acid methyl ether | 21.11 | 10.59 |
| Tyrothricin | 22.36 | 0.75 |
| Hexachlorophene | 23.92 | 0.26 |
| Cefamandole sodium | 23.97 | 2.07 |
| Echinocystic acid | 24.64 | - |
| (-)-Deguelin | 27.97 | 1.67 |
| Dihydrogambogic acid | 30.40 | 1.39 |
| Oligomycin (A shown) | 33.87 | 0.26 |
| Niclosamide | 35.45 | 0.08 |
| Gossypol-acetic acid complex | 36.14 | 3.10 |
| Methyl deoxycholate | 36.71 | 4.84 |
| Dihydrorotenone | 37.31 | 1.11 |
| Gramicidin | 37.54 | 7.59 |

(continued)

| Compound | Uptake of tauro-nor-THCA-24-DBD (% of control) | $K_i$ (μM) |
|---|---|---|
| Cholic acid, methyl ester | 37.90 | 2.32 |
| Temefos | 38.51 | 4.16 |
| Scandenin | 38.92 | - |
| Garcinolic acid | 42.42 | 5.14 |
| Nicardipine hydrochloride | 42.69 | 3.53 |
| Geneticin | 43.81 | - |
| Flufenamic acid | 44.12 | 7.51 |
| Dehydroavocatin-1 -acetate | 44.14 | 118.11 |
| Lanatoside c | 45.13 | 125.07 |
| Crustecdysone | 46.18 | - |
| Pentachlorophenol | 46.29 | 2.27 |
| (+)- Linalool | 46.35 | - |
| Irigenin, 7-benzyl ether | 46.71 | 13.00 |
| Citropten | 46.97 | - |
| Phenylalanyltyrosine | 47.22 | - |
| Rauwolscine hydrochloride | 47.23 | 39.31 |
| Diethylstilbestrol | 48.51 | 1.57 |
| Nimodipine | 48.65 | 8.38 |
| Haematommic acid | 49.33 | - |
| G786-1037 | 49.46 | - |
| 5-nitro-2-phenylpropylaminobenzoic acid | 49.55 | 0.82 |
| Xanthopterin | 49.67 | - |
| Tubocurarine chloride | 49.76 | - |

Figure 3 shows the uptake of 3 μM tauro-nor-THCA-24-DBD in NTCP-transfected CHO cells in the presence of equimolar concentrations of each compound (n = 2556). Uptake is expressed relative to the control, i.e. uptake of 3 μM tauro-nor-THCA-24-DBD in absence of any compound. Each compound was tested in triplicate. The x-axis shows the number of compounds and the y-axis shows the tauro-nor-THCA-24-DBD uptake in % of the control group.

Example 4: Chemometric modelling. Overlay model

The best overlay model obtained from the training compounds is depicted in Figure 4. It included the compounds dihydrorotenone, pentachlorophenol, gossypol and dihydrogambogic acid. Notably, the most active compound (niclosamide) was not included. The selected overlay was formed by a core of rings mainly surrounded by hydrogen bond donor and acceptor groups, but hydrophobic groups were also present. As can be seen from Figure 4B most of the hydrogen bonding groups were predicted to be exposed to the solvent and many of the hydrophobic groups are buried inside the taurocholate binding site. The superimposition of the proposed overlay with the NTCP taurocholate binding site is consistent with the observed binding mode of taurocholate to the NTCP homolog receptor ASBT from N. meningitidis. These observations support the predicted binding poses of the most active NTCP inhibitors found in this study.

Figure 4 shows the best overlay model of the most active NTCP inhibitors (A) and superimposition of the overlay in the taurocholate binding site of NTCP (B). Hydrogen bond acceptor and donor groups are represented as red and blue meshes, respectively. Rings are depicted in solid green, hydrophobic groups in solid yellow spheres and anions in solid red spheres.

To validate the predicted overlay, it was challenged to retrieve the nine NTCP inhibitors on the VSVS from a database also containing 2462 non-inhibitors. The accumulative curve produced by the ligands overlay in the virtual screening of the VSVS is shown in Figure 5. It can be observed that the virtual screening performance of the selected model is away from random. More specifically, the selected model achieved an area under the ROC curve of 0.73, EF 0.5% of 21.12, EF1% of 10.98 and EF 5% of 8.86. Hence, the proposed model was able to retrieve over 20 times more NTCP inhibitors in the first 0.5% of screened data than would be expected from a uniform distribution of the inhibitors on the whole VSVS (one inhibitor every 275 compounds). The selected model retrieved four out of nine inhibitors in the first 5% of screened data (124 compound). The results of the virtual screening validation of the obtained ligands overlay suggested that this model was effective in the virtual screening of databases of chemical compounds in the search for NTCP inhibitors.

Figure 5 shows the accumulative curve of the virtual screening validation set with the selected ligands overlay (solid line). The dashed line represents classification according to a random process. The x-axis shows the fraction of non-inhibitors retrieved and the y-axis shows the fraction of inhibitors retrieved.

ROCS overlays are based on shape and chemical features, so we evaluated the virtual screening performance of these features alone. This analysis revealed low performance of the models, indicating that neither of them was sufficient to correctly separate NTCP inhibitors and non-inhibitors. Instead, both shape and chemical features must be considered jointly by means of the TanimotoCombo metric for correct classification of NTCP inhibitors. We also evaluated the performance of the ligand overlay found when no receptor information was included in the ligand-based modeling strategy herein presented. The results of this evaluation led to a model with no virtual screening performance (data not shown). Hence, our results pointed to the need of incorporating receptor information through molecular docking, for better performing models in the virtual screening of NTCP inhibitors.

<u>Example 5: Chemometric modelling. Structure-based model</u>

A model for the structure-based VS of NTCP inhibitors was also developed. The performance of the obtained CompScore models is summarized in Table 3. Dock and Gold outperformed the results obtained with Fred as primary docking software. Despite achieving the same performance, the standard deviation of BEDROC obtained with Gold was lower than that obtained with Dock. Furthermore, the CompScore model derived from the use of Gold as primary docking program was simpler than the one obtained with Dock. These last observations led to the selection of the Gold model for the structure-based virtual screening of potential NTCP inhibitors. The selected CompScore model consisted of the primary docking of compounds to the NTCP homology model with Gold and the rescoring of the predicted ligand poses with scoring functions of the three docking software packages. The list of scoring function components included in the selected CompScore model is provided as Supplementary Information. The model was challenged to predict the 100 external datasets generated during the data portioning process and achieved a BEDROC value of 0.25 ± 0.05. The latter did not differ from the initial enrichment obtained during the training of the models. This indicated that, in addition of showing performance away from random (BEDROC = 0), the selected CompScore model was predictive.

*Table 3: Performance summary for the trained CompScore models.*

| | Primary docking | | |
|---|---|---|---|
| | Fred | Dock | Gold |
| Mean ± SD BEDROC[(a)] | 0.13 ± 0.013 | 0.26 ± 0.021 | 0.26 ± 0.018 |
| Number of SFC[(b)] | 12 | 19 | 13 |
| [(a)]Mean ± SD BEDROC ($\alpha$ = 160.9) across the 100 trained models. [(b)]Number of scoring functions components in more than 50 models. BEDROC, Boltzmann-enhanced discrimination of receiver operating characteristic | | | |

<u>Example 6: Chemometric modelling. Virtual screening</u>

The ROCS overlay model was used as a first filter to screen the 1 870 461 compounds in v25 of the ChEMBL database. The 10 000 top ranked chemicals (~0.5% of ChEMBL) were selected for further analysis with the CompScore model. The TanimotoCombo score of these top ranked molecules ranged from 1.168 to 0.328, with a median of 0.344. These 10 000 selected compounds were subsequently docked into the NTCP binding site and rescored using the previously validated CompScore model. From the docked and rescored compounds, 100 top ranked compounds (1%) were selected as the most probable NTCP inhibitors. This final subset of compounds was clustered and their predicted binding poses to NTCP were visually inspected. The ChEMBL IDs of these potential NTCP inhibitors are provided as Supplementary Information.

Example 7: Chemometric modelling. Prospective validation

Baohuoside I, fukugetin and sabutoclax showed concentration-dependent inhibition of tauro-nor-THCA-24-DBD uptake in NTCP-transfected CHO cells (Figure 6). Their respective $K_i$ values were 12.6 ± 4.1, 23.8 ± 4.6 and 0.522 ± 0.077 μM. Nepitrin, quercetin and sennoside B did not show concentration-dependent inhibition.

Figure 6 shows the concentration-dependent inhibition of 3 μM tauro-nor-THCA-24-DBD in the presence of multiple concentrations of baohuoside I (A), fukugetin (B) and sabutoclax (C). Points represent mean (± SD) of triplicate experiments. The line represents the best fit of the experimental data according to the sigmoid Emax model. The x-axis shows the concentration of NCTP inhibitor in μM and the y-axis shows the tauro-nor-THCA-24-DBD in pmol·mg protein$^{-1}$·min$^{-1}$.

Example 8: Assay performance

The median Z score over all experiments was 0.5640 (interquartile range 0.3940 to 0.7101) The uptake of the controls in NTCP-transfected and wild-type cells is depicted in Figure 7. A one-sided Dunnett's test did not show interference of any compound with the excitation or emission wavelengths of tauro-nor-THCA-24-DBD (Table 4).

*Table 4: Interference of any compound with the excitation or emission wavelengths of tauro-nor-THCA-24-DBD.*

| Compound and concentration | p value | Compound and concentration | p value |
|---|---|---|---|
| 5-nitro-2-phenylpropylaminobenzoic acid [nppb] 0.05 μM | 0,992534 | Gossypol 5 μM | 1 |
| 5-nitro-2-phenylpropylaminobenzoic acid [nppb] 0.5 μM | 1 | Gramicidin 0.05 μM | 0,996738 |
| 5-nitro-2-phenylpropylaminobenzoic acid [nppb] 5 μM | 1 | Gramicidin 0.5 μM | 1 |
| Baohuoside 0.05 μM | 1 | Gramicidin 5 μM | 0,999399 |
| Baohuoside 0.5 μM | 1 | Hexachlorophene 0.05 μM | 1 |
| Baohuoside 5 μM | 1 | Hexachlorophene 0.5 μM | 1 |
| Bosentan 0.05 μM | 0,999961 | Hexachlorophene 5 μM | 1 |
| Bosentan 0.5 μM | 1 | Irigenin, 7-benzyl ether 0.05 μM | 1 |
| Bosentan 5 μM | 1 | Irigenin, 7-benzyl ether 0.5 μM | 0,999874 |
| Bromosulfalein 0.05 μM | 1 | Irigenin, 7-benzyl ether 5 μM | 1 |
| Bromosulfalein 0.5 μM | 0,999977 | Lanatoside C 0.05 μM | 1 |
| Bromosulfalein 5 μM | 1 | Lanatoside C 0.5 μM | 1 |
| Candesartan 0.05 μM | 1 | Lanatoside C 5 μM | 1 |
| Candesartan 0.5 μM | 1 | Lobaric acid 0.05 μM | 1 |
| Candesartan 5 μM | 0,999943 | Lobaric acid 0.5 μM | 1 |
| Candesartan cilextil 0.05 μM | 1 | Lobaric acid 5 μM | 1 |
| Candesartan cilextil 0.5 μM | 0,922233 | Methyldeoxycholate 0.05 μM | 1 |
| Candesartan cilextil 5 μM | 1 | Methyldeoxycholate 0.5 μM | 1 |
| Cefamandol 0.05 μM | 1 | Methyldeoxycholate 5 μM | 1 |
| Cefamandol 0.5 μM | 1 | Nicardipine 0.05 μM | 1 |
| Cefamandol 5 μM | 1 | Nicardipine 0.5 μM | 1 |
| Cholic acid, methyl ester 0.05 μM | 1 | Nicardipine 5 μM | 1 |
| Cholic acid, methyl ester 0.5 μM | 1 | Niclosamide 0.05 μM | 1 |

(continued)

| Compound and concentration | p value | Compound and concentration | p value |
|---|---|---|---|
| Cholic acid, methyl ester 5 μM | 1 | Niclosamide 0.5 μM | 1 |
| Cyclosporin A 0.05 μM | 1 | Niclosamide 5 μM | 1 |
| Cyclosporin A 0.5 μM | 1 | Nimodipine 0.05 μM | 1 |
| Cyclosporin A 5 μM | 1 | Nimodipine 0.5 μM | 1 |
| Deguelin 0.05 μM | 1 | Nimodipine 5 μM | 0,999984 |
| Deguelin 0.5 μM | 1 | Oligomycin (a shown) 0.05 μM | 1 |
| Deguelin 5 μM | 1 | Oligomycin (a shown) 0.5 μM | 1 |
| Dehydroavocatin-1-acetate 0.05 μM | 1 | Oligomycin (a shown) 5 μM | 1 |
| Dehydroavocatin-1-acetate 0.5 μM | 0,999603 | Pentachlorophenol 0.05 μM | 1 |
| Dehydroavocatin-1-acetate 5 μM | 1 | Pentachlorophenol 0.5 μM | 0,930691 |
| Diethylstilbestrol 0.05 μM | 0,999221 | Pentachlorophenol 5 μM | 1 |
| Diethylstilbestrol 0.5 μM | 1 | Rauwolscine hydrochloride 0.05 μM | 1 |
| Diethylstilbestrol 5 μM | 1 | Rauwolscine hydrochloride 0.5 μM | 1 |
| Dihydrogambogic acid 0.05 μM | 1 | Rauwolscine hydrochloride 5 μM | 1 |
| Dihydrogambogic acid 0.5 μM | 1 | Rifampicin 0.05 μM | 1 |
| Dihydrogambogic acid 5 μM | 0,085908 | Rifampicin 0.5 μM | 1 |
| Dihydrorotenone 0.05 μM | 0,999995 | Rifampicin 5 μM | 1 |
| Dihydrorotenone 0.5 μM | 1 | Ritonavir 0.05 μM | 1 |
| Dihydrorotenone 5 μM | 1 | Ritonavir 0.5 μM | 1 |
| Erythromycin estolate 0.05 μM | 1 | Ritonavir 5 μM | 1 |
| Erythromycin estolate 0.5 μM | 1 | Robustic acid methyl ether 0.05 μM | 1 |
| Erythromycin estolate 5 μM | 1 | Robustic acid methyl ether 0.5 μM | 1 |
| Flufenamic acid 0.05 μM | 1 | Robustic acid methyl ether 5 μM | 1 |
| Flufenamic acid 0.5 μM | 1 | Sabutoclax 0.05 μM | 1 |
| Flufenamic acid 5 μM | 1 | Sabutoclax 0.5 μM | 1 |
| Fukugetin 0.05 μM | 1 | Sabutoclax 5 μM | 1 |
| Fukugetin 0.5 μM | 1 | Tannic acid 0.05 μM | 1 |
| Fukugetin 5 μM | 1 | Tannic acid 0.5 μM | 1 |
| G786-1037 0.05 μM | 1 | Tannic acid 5 μM | 1 |
| G786-1037 0.5 μM | 1 | Temefos 0.05 μM | 0,999959 |
| G786-1037 5 μM | 1 | Temefos 0.5 μM | 1 |
| Garcinolic acid 0.05 μM | 1 | Temefos 5 μM | 0,999976 |
| Garcinolic acid 0.5 μM | 1 | Tyrothricin 0.05 μM | 1 |

(continued)

| Compound and concentration | p value | Compound and concentration | p value |
|---|---|---|---|
| Garcinolic acid 5 μM | 1 | Tyrothricin 0.5 μM | 1 |
| Gossypol 0.05 μM | 1 | Tyrothricin 5 μM | 1 |
| Gossypol 0.5 μM | 1 | | |

Figure 7 shows the uptake of tauro-nor-THCA-24-DBD in NTCP-transfected and wild-type CHO cells throughout the experiments over time as a measure of quality control. Solid lines represent the mean and dashed lines the SD of the overall uptake in the respective cell type. The x-axis shows the experiment number and the y-axis shows the tauro-nor-THCA-24-DBD uptake in pmol·mg $protein^{-1}\cdot min^{-1}$.

The present invention is by no means limited to the above described preferred embodiments and/or experiments thereof. The rights sought are defined by the following claims within the scope of which many modifications can be envisaged.

## Claims

**1.** Compound according to formula (I), or a stereoisomer, tautomer, racemic, salt, hydrate, N-oxide form, or solvate thereof, in particular for use in the prevention and/or treatment and/or cure of hepatitis B virus infection,

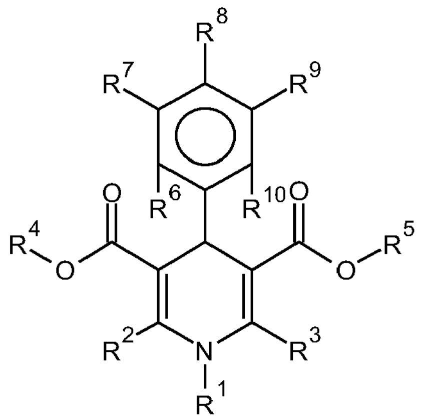

(I)

wherein:

$R^1$ is selected from the group of H, $C_1$-$C_6$ alkyl, $C_1$-$C_6$ alkene, $C_1$-$C_6$ alkyne, -$NR^{11}R^{12}$, -$OR^{13}$, $Ar^1$, $HetAr^1$,

wherein said $C_1$-$C_6$ alkyl, $C_1$-$C_6$ alkene,or $C_1$-$C_6$ alkyne is optionally substituted with one or more $Ar^3$, and wherein said $Ar^1$ or $HetAr^1$ is optionally substituted with one or more selected from the group of -$NR^{11}R^{12}$, -$OR^{13}$,

wherein each occurrence of $R^{11}$, $R^{12}$ and $R^{13}$ is independently selected from the group of H, $C_1$-$C_6$ alkyl, $C_1$-$C_6$ alkene, $C_1$-$C_6$ alkyne;

$R^2$ and $R^3$ are independently selected from the group of H, $C_1$-$C_6$ alkyl, $C_1$-$C_6$ alkene, $C_1$-$C_6$ alkyne, -$NR^{14}R^{15}$ , -$OR^{16}$,

wherein each occurence of $R^{14}$, $R^{15}$ and $R^{16}$ is independently selected from the group of H, $C_1$-$C_6$ alkyl, $C_1$-$C_6$ alkene, $C_1$-$C_6$ alkyne;

$R^4$ and $R^5$ are independently selected from the group of H, $C_1$-$C_6$ alkyl, $C_1$-$C_6$ alkene, $C_1$-$C_6$ alkyne, -$NR^{17}R^{18}$, $Ar^2$, $HetAr^2$, $Het^1$,

wherein said $C_1$-$C_6$ alkyl, $C_1$-$C_6$ alkene, $C_1$-$C_6$ alkyne, $Ar^2$, $HetAr^2$ and/or $Het^1$ are optinally substituted with

one or more selected from the group of $C_1$-$C_6$ alkyl, $C_1$-$C_6$ alkene, $C_1$-$C_6$ alkyne, - $NR^{19}R^{20}$, $-OR^{21}$, $Ar^2$, $HetAr^2$, $Het^1$, and carbonylaldehyde; wherein each of said $C_1$-$C_6$ alkyl, $C_1$-$C_6$ alkene,and $C_1$-$C_6$ alkyne are optionally substituted with one ore more $Ar^4$;
wherein each occurence of $R^{17}$, $R^{18}$, $R^{19}$, $R^{20}$, and $R^{21}$ is independently selected from the group of H, $C_1$-$C_6$ alkyl, $C_1$-$C_6$ alkene, $C_1$-$C_6$ alkyne and carbonyl, wherein each of said $C_1$-$C_6$ alkyl, $C_1$-$C_6$ alkene,and $C_1$-$C_6$ alkyne are optionally substituted with one or more $Ar^5$;

$R^6$, $R^7$, $R^8$, $R^9$, and $R^{10}$ are independently selected from the group of H, $NO_2$, halo, CN, carbonyl

wherein $HetAr^1$ and $HetAr^2$ are each independently a 3- to 10-membered aromatic heterocycle optionally comprising 1 to 3 heteroatoms selected from O, N and S;
wherein $Ar^1$, $Ar^2$ , $Ar^3$, $Ar^4$ and $Ar^5$ are each independently a 3- to 10-membered aromatic cycle optionally comprising 1 to 3 heteroatoms selected from O, N and S; and
wherein $Het^1$ is a 3- to 10-membered heterocycle having from 1 to 3 heteroatoms selected from O, N and S.

**2.** Compound for use according to claim 1, wherein $R^6$, $R^7$, $R^8$, $R^9$, and $R^{10}$ are independently selected from the group of H, $NO_2$.

**3.** Compound for use according to any one of the preceding claims, wherein $R^7$ and/or $R^9$ are $NO_2$.

**4.** Compound for use according to any one of the preceding claims, wherein $R^7$ and/or $R^9$ are $NO_2$ and wherein $R^6$, $R^8$, and $R^{10}$ are H.

**5.** Compound for use according to any one of the preceding claims, wherein formula (I) is formula (Ia) or formula (Ib)

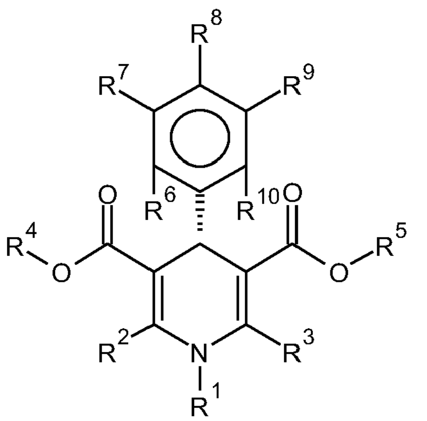
(Ia)

or

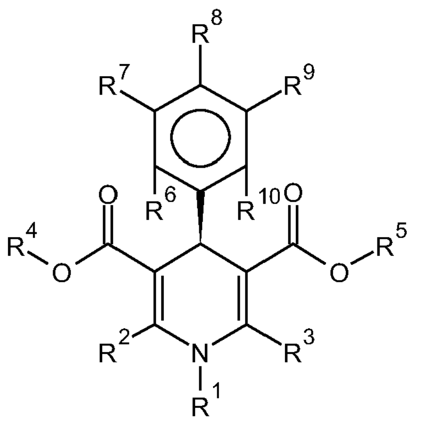
(Ib)

wherein $R^1$, $R^2$, $R^3$, $R^4$, $R^5$, $R^6$, $R^7$, $R^8$, $R^9$, and $R^{10}$ are defined according to any one of the preceeding claims.

**6.** Compound for use according to any one of the preceding claims, wherein $Ar^1$ and $Ar^3$ independently selected from the group of cyclopentadiene, phenyl, naphthyl, cycloheptatriene, and cyclooctatetraene; in particular $Ar^1$ and $Ar^3$ represent phenyl.

**7.** Compound for use according to any one of the preceding claims, wherein $Ar^2$, $Ar^4$ and $Ar^5$ and the optionally substituted $Ar^2$, $Ar^4$ and $Ar^5$ are each independently selected from the group of cyclopentadiene, phenyl, tolyl, xylyl, naphthyl, cycloheptatriene, cyclooctatetraene.

**8.** Compound for use according to any one of the preceding claims, wherein $HetAR^1$ and $HetAR^2$ are independently selected from the group of azirine, oxirene, thiirene, diazirine, azete, oxete, thiete, pyrrole, furan, thiophene, imidazole, oxathiole, oxazole, thiazole, triazole, furazan, thiadiazole, dioxazole, dithiazole, tetrazole, pyridine, pyran, thiopyran, diazine, oxazine, thiazine, dioxine, dithiin, triazine.

**9.** Compound for use according to any one of the preceding claims, wherein $Het^1$ is independently selected from the

group of aziridine, oxirane, thiirane, diaziridine, oxaziridine, azetidine, oxetane, thietane, pyrrolidine, tetrahydrofuran, tetrahydrothiophene, imidazolidine, oxathiolidine, oxazolidine, thiazolidine, dioxolane, dithiolane, piperidine, oxane, thiane, diazinane, morpholine, thiomorpholine, dioxane, dithiane, triazinane, trioxane, trithiane.

**10.** Pharmaceutical composition according to any one of the preceding claims, wherein $R^1$ is selected from the group of: H, $C_1$-$C_4$ alkyl,

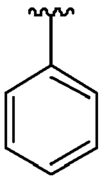 , 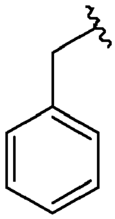 , 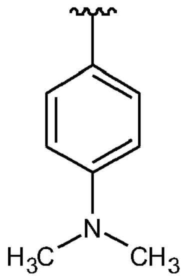 .

**11.** Compound for use according to any one of the preceding claims, wherein $R^2$ and $R^3$ are independently selected from the group of H, $C_1$-$C_4$ alkyl, $NH_2$, preferably wherein $R^2$ and/or $R^3$ are $-CH_3$, even more preferably wherein wherein $R^2$ and $R^3$ are the same.

**12.** Compound for use according to any one of the preceding claims, wherein $R^4$ and $R^5$ are independently selected from the group of: $C_1$-$C_4$ alkyl, $C_1$-$C_4$ alkene ,

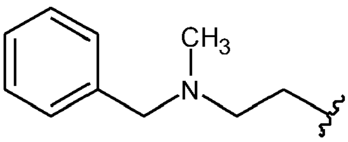 , 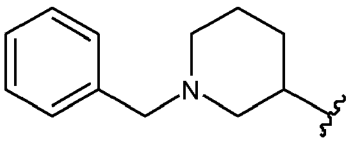 ,

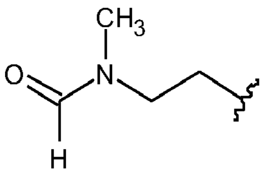 , 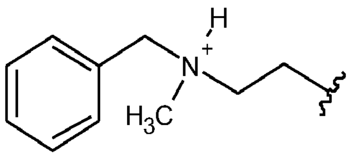 , 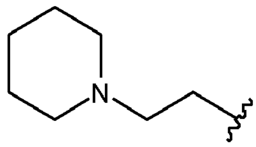 ,

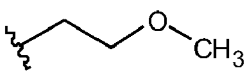 , 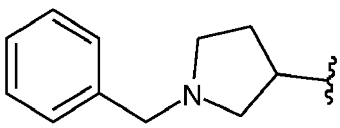 , 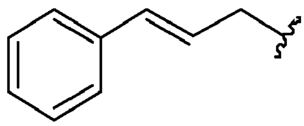 ,

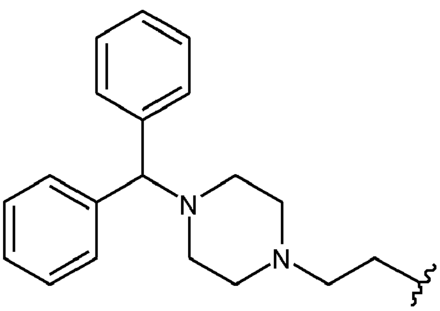 , 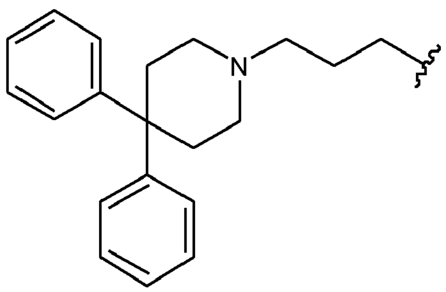 ,

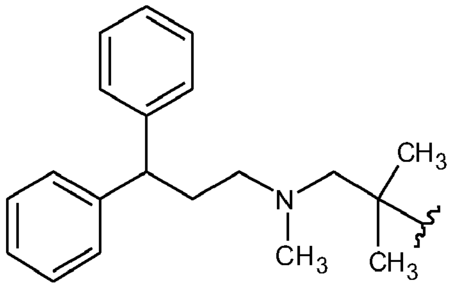
,
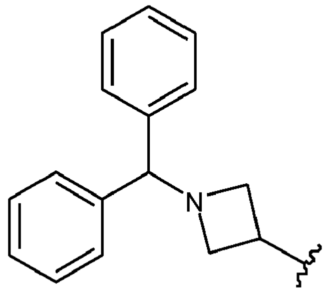

**13.** Compound for use according to any one of the preceding claims, wherein $R^4$ and $R^5$ are the same.

**14.** Pharmaceutical composition comprising a compound according to any one of the preceding claims, optionally further comprises a pharmaceutical acceptable carrier.

**15.** Pharmaceutical composition according to claim 14, for use in human or veterinary medicine.

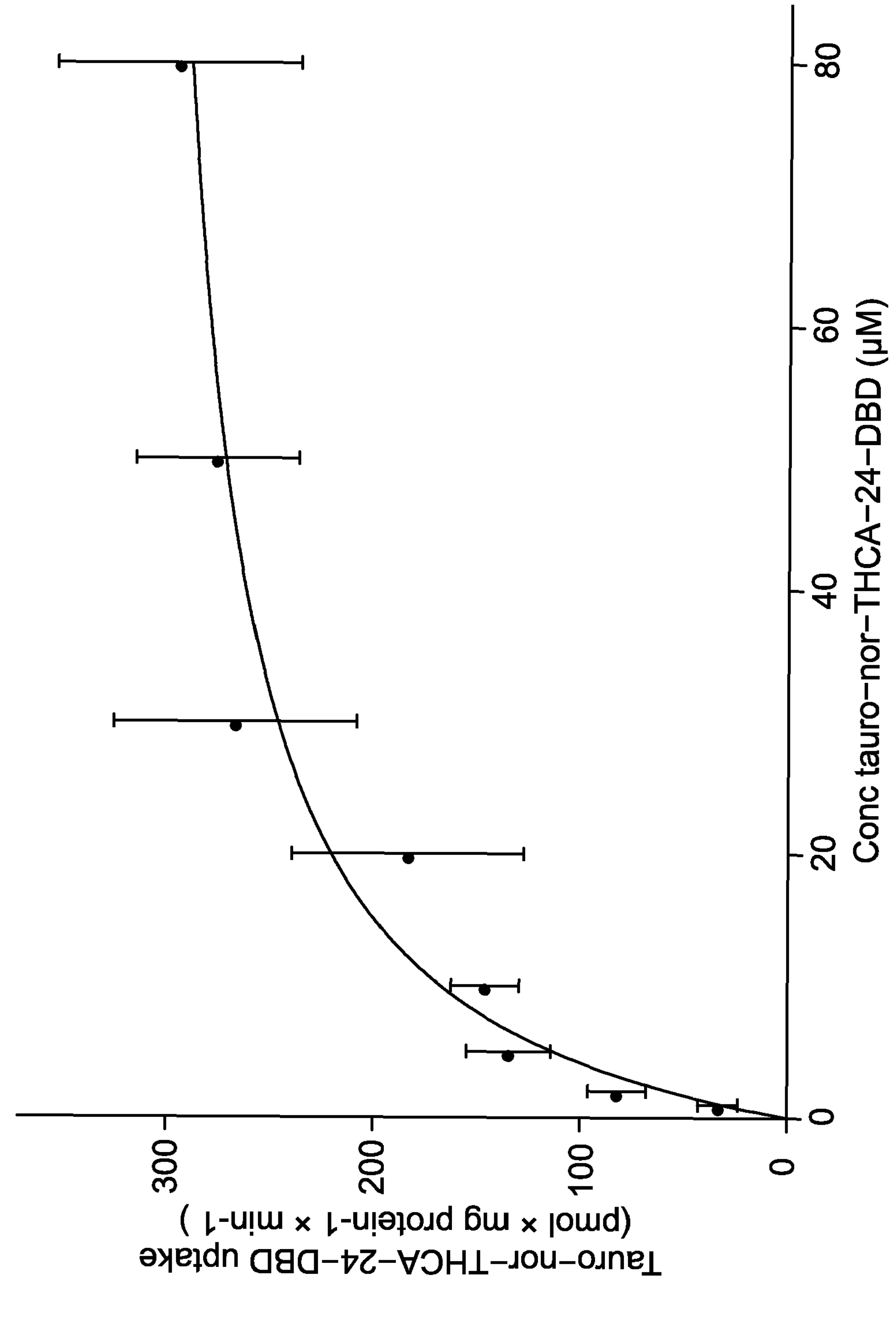
Tauro-nor-THCA-24-DBD uptake
(pmol × mg protein-1 × min-1 )
0
100
200
300
0
20
40
60
80
Conc tauro-nor-THCA-24-DBD (µM)

FIG. 1

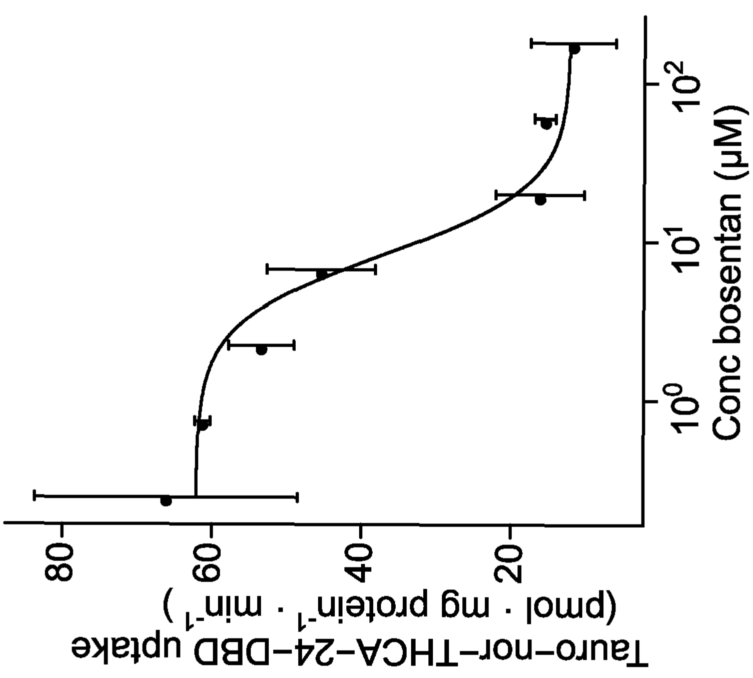
Tauro-nor-THCA-24-DBD uptake
(pmol · mg protein-1 · min-1 )
80
60
40
20
10^0
10^1
10^2
Conc bosentan (µM)

FIG. 2A

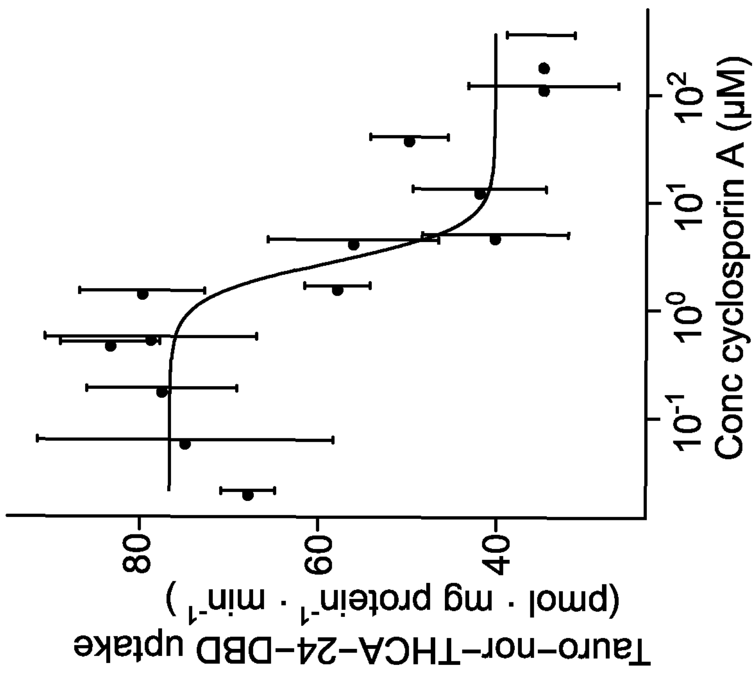
Tauro-nor-THCA-24-DBD uptake
(pmol · mg protein-1 · min-1 )
80
60
40
10^-1
10^0
10^1
10^2
Conc cyclosporin A (µM)

FIG. 2B

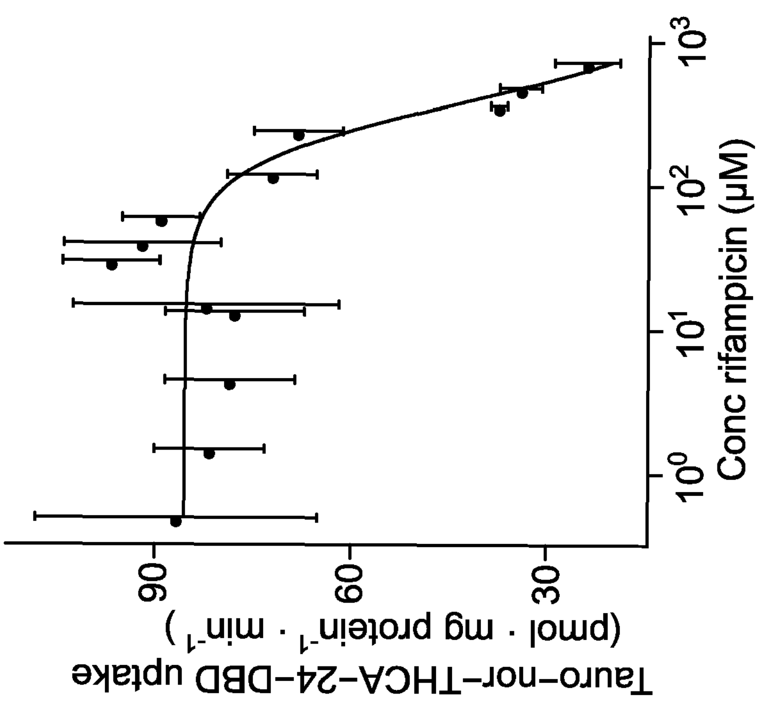
Tauro–nor–THCA–24–DBD uptake
(pmol · mg protein⁻¹ · min⁻¹ )
90
60
30
10⁰
10¹
10²
10³
Conc rifampicin (µM)

FIG. 2C

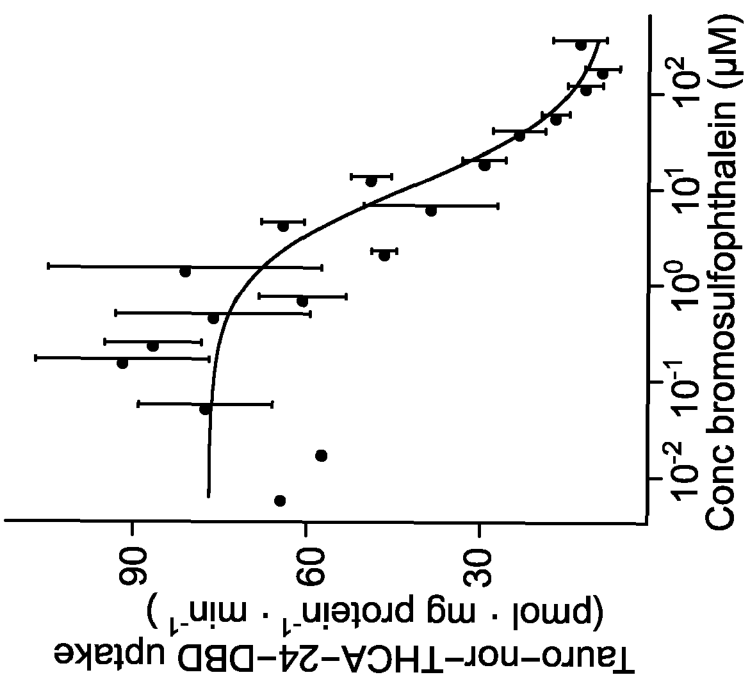
Tauro–nor–THCA–24–DBD uptake
(pmol · mg protein⁻¹ · min⁻¹ )
90
60
30
10⁻²
10⁻¹
10⁰
10¹
10²
Conc bromosulfophthalein (µM)

FIG. 2D

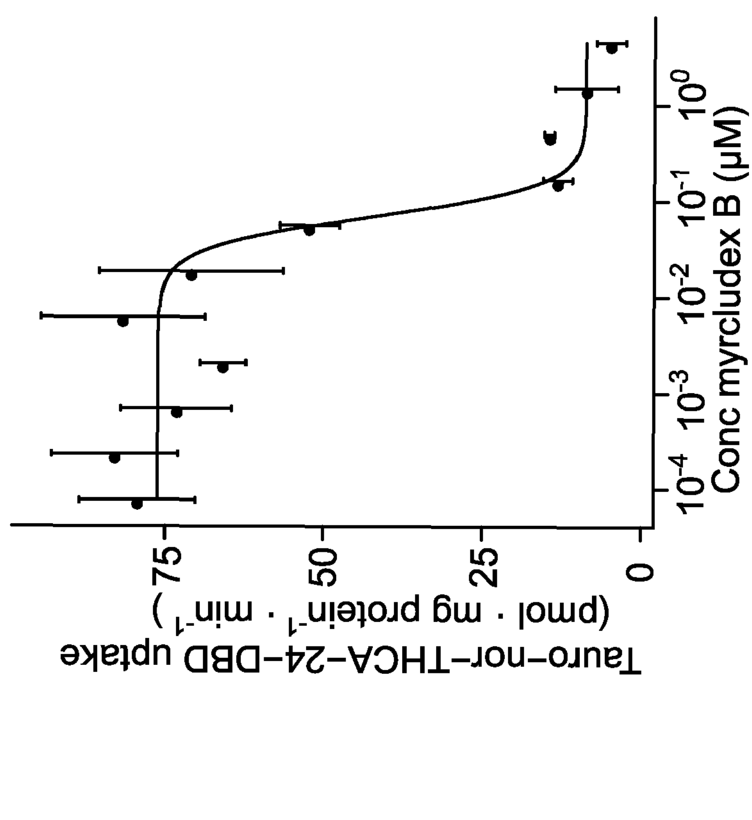
Tauro−nor−THCA−24−DBD uptake
(pmol · mg protein$^{-1}$ · min$^{-1}$)
0
25
50
75
$10^{-4}$
$10^{-3}$
$10^{-2}$
$10^{-1}$
$10^{0}$
Conc myrcludex B (μM)

FIG. 2E

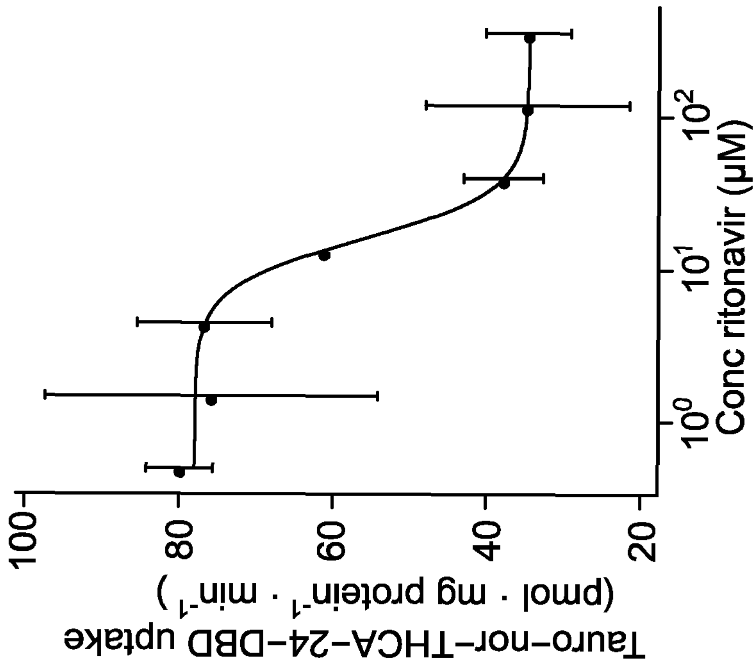
Tauro−nor−THCA−24−DBD uptake
(pmol · mg protein$^{-1}$ · min$^{-1}$)
20
40
60
80
100
$10^{0}$
$10^{1}$
$10^{2}$
Conc ritonavir (μM)

FIG. 2F

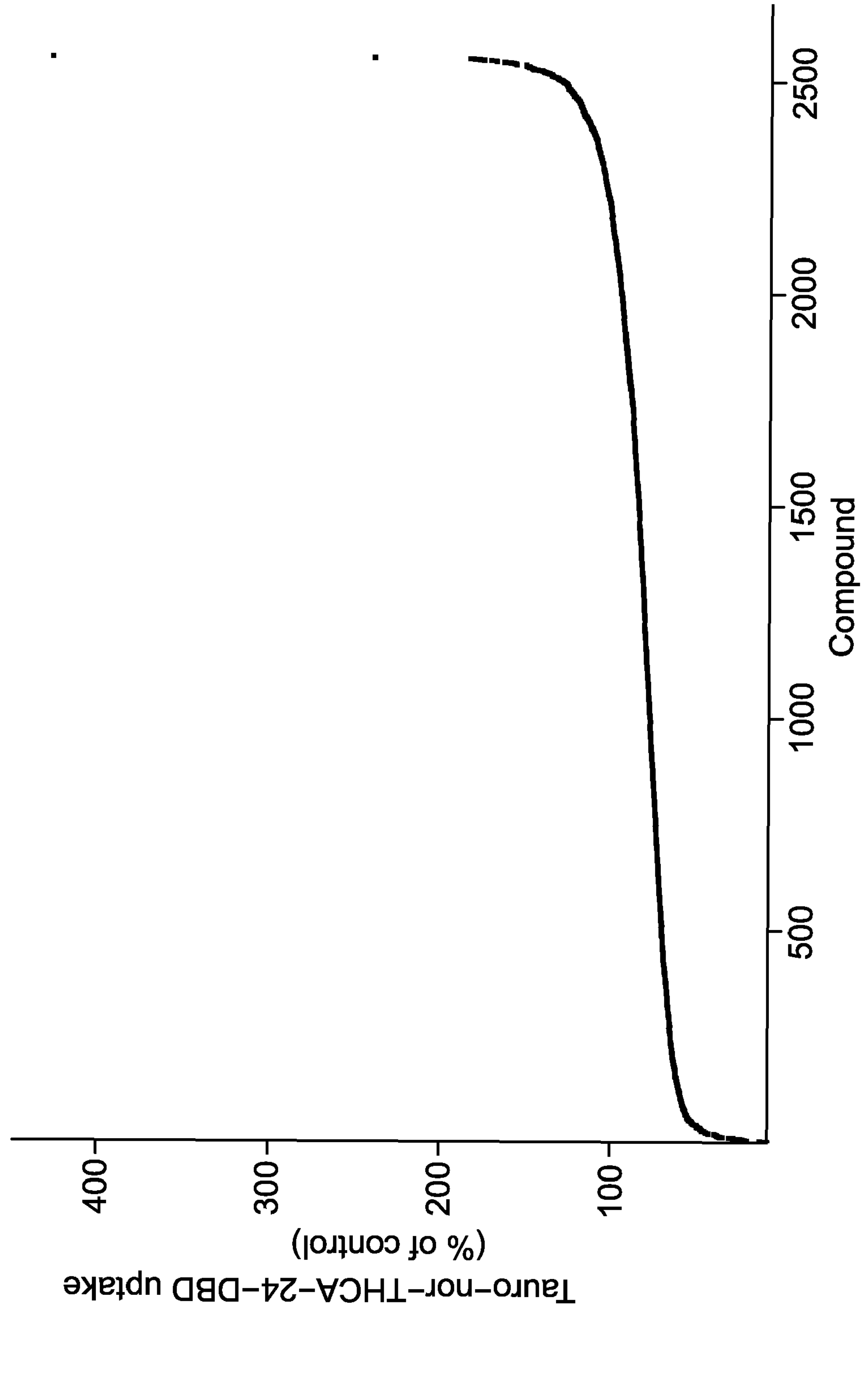
Tauro-nor-THCA-24-DBD uptake
(% of control)
100
200
300
400
Compound
500
1000
1500
2000
2500

FIG. 3

FIG. 4A

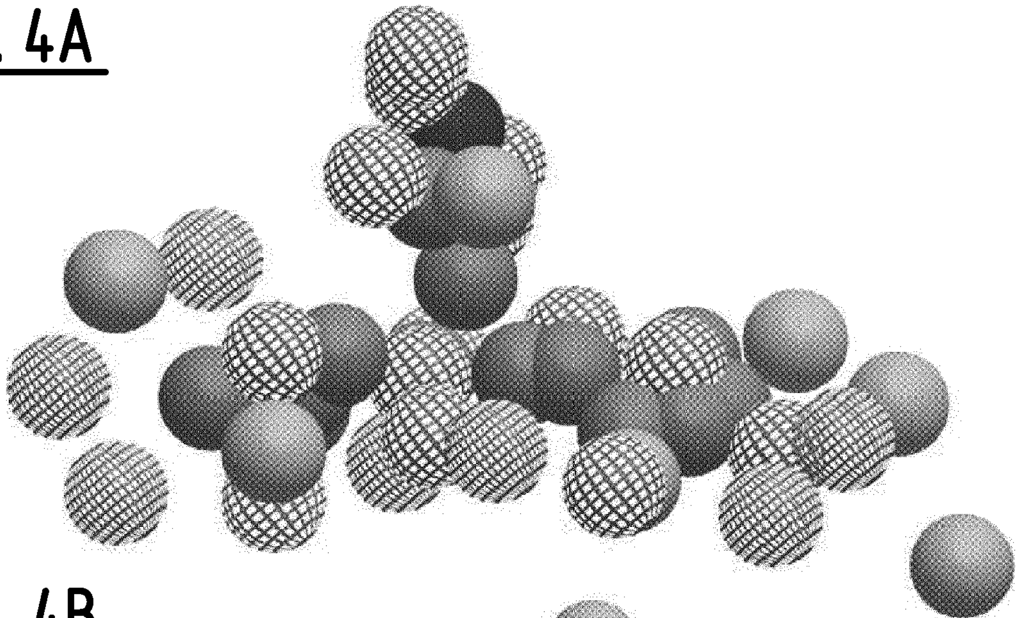

FIG. 4B

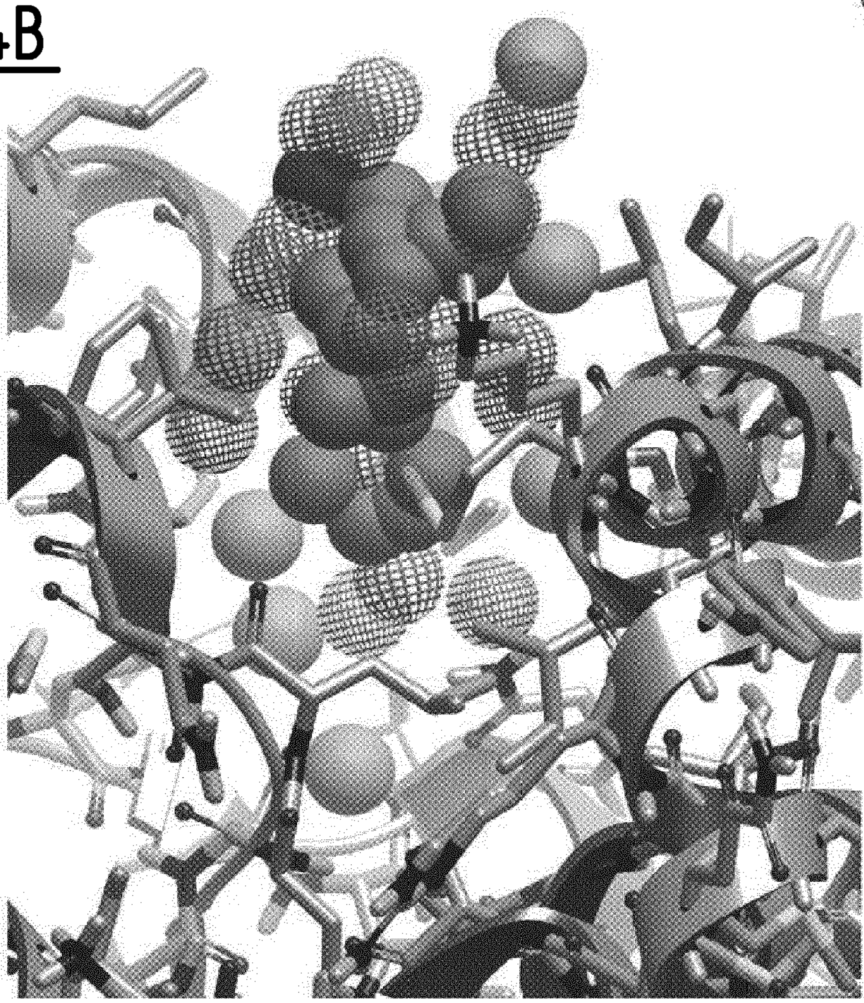

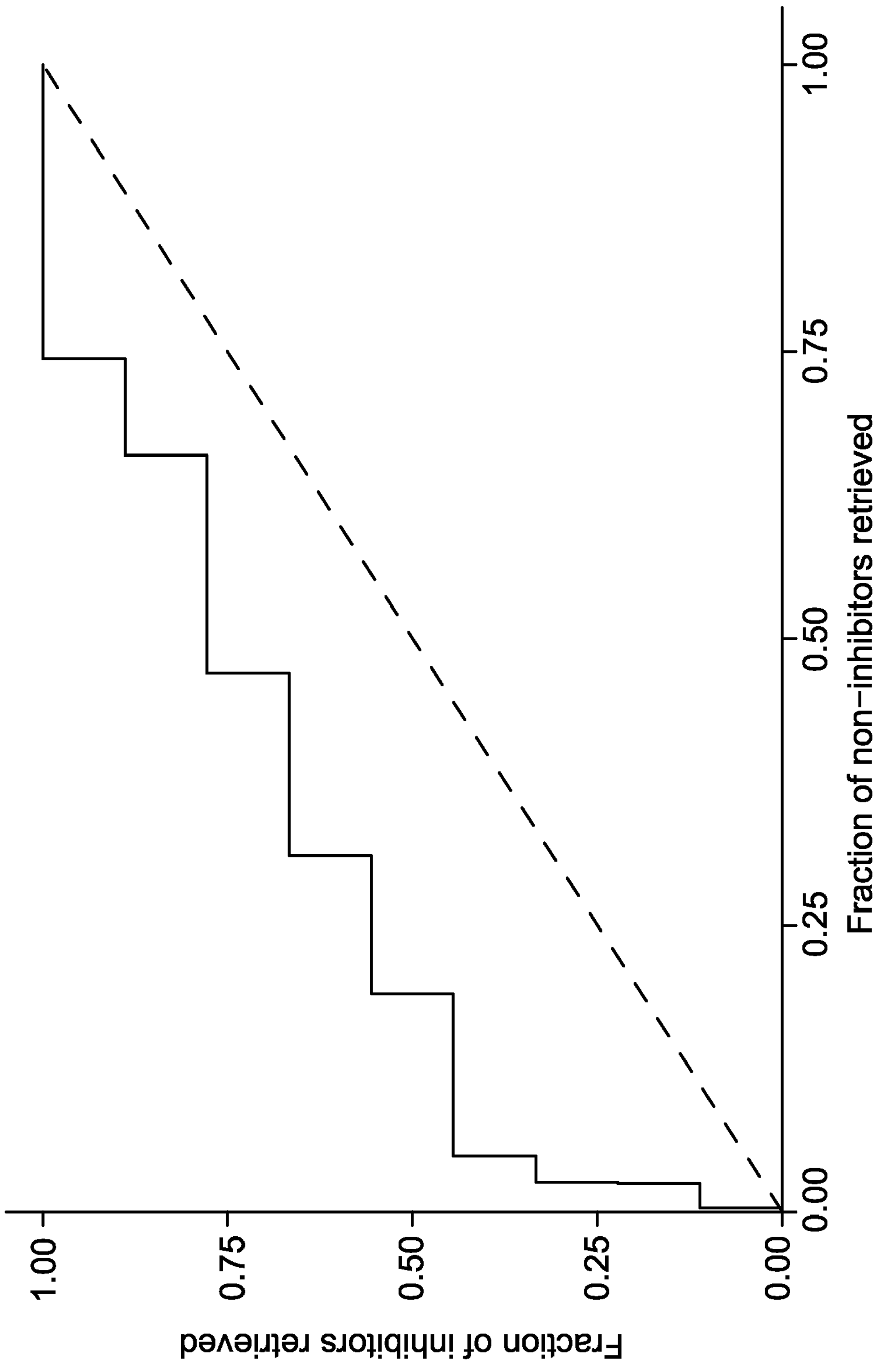
Fraction of inhibitors retrieved
1.00
0.75
0.50
0.25
0.00
0.00
0.25
0.50
0.75
1.00
Fraction of non−inhibitors retrieved

FIG. 5

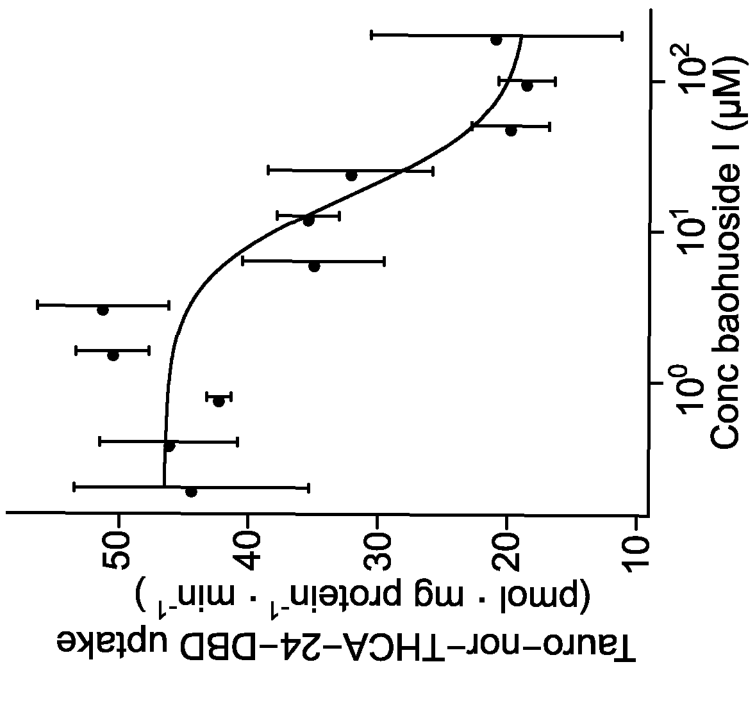
Tauro−nor−THCA−24−DBD uptake
(pmol · mg protein$^{-1}$ · min$^{-1}$)
50
40
30
20
10
$10^0$
$10^1$
$10^2$
Conc baohuoside I (μM)

FIG. 6A

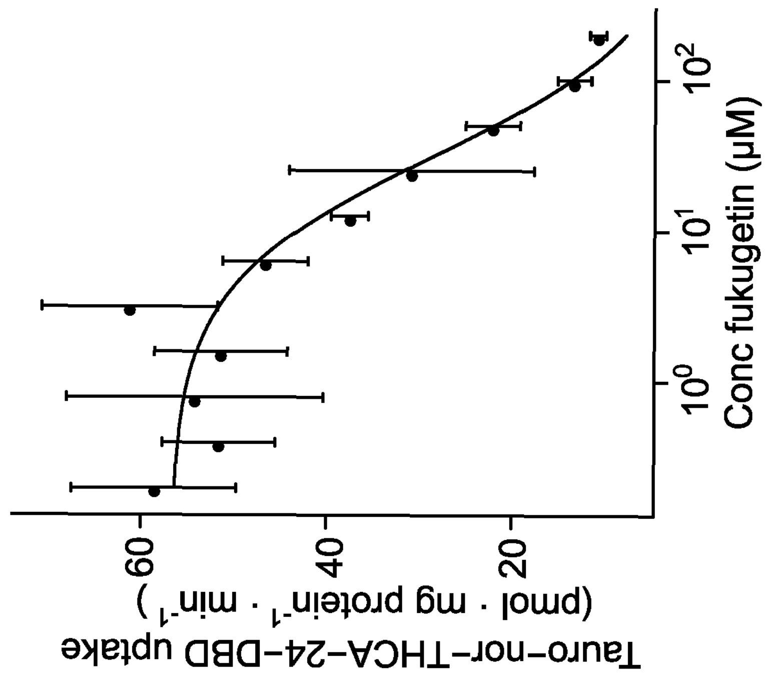
Tauro−nor−THCA−24−DBD uptake
(pmol · mg protein$^{-1}$ · min$^{-1}$)
60
40
20
$10^0$
$10^1$
$10^2$
Conc fukugetin (μM)

FIG. 6B

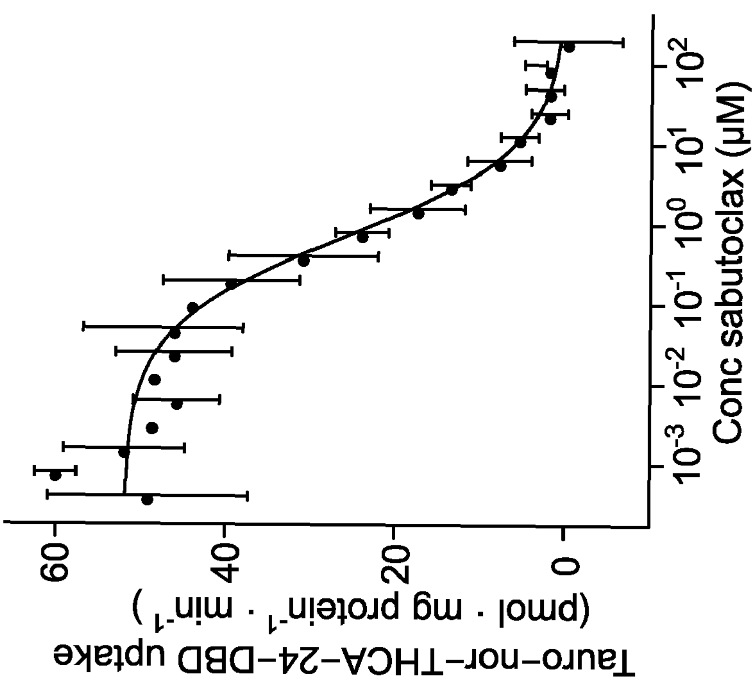
Tauro-nor-THCA-24-DBD uptake
(pmol · mg protein$^{-1}$ · min$^{-1}$)
60
40
20
0
$10^{-3}$ $10^{-2}$ $10^{-1}$ $10^{0}$ $10^{1}$ $10^{2}$
Conc sabutoclax (µM)

FIG. 6C

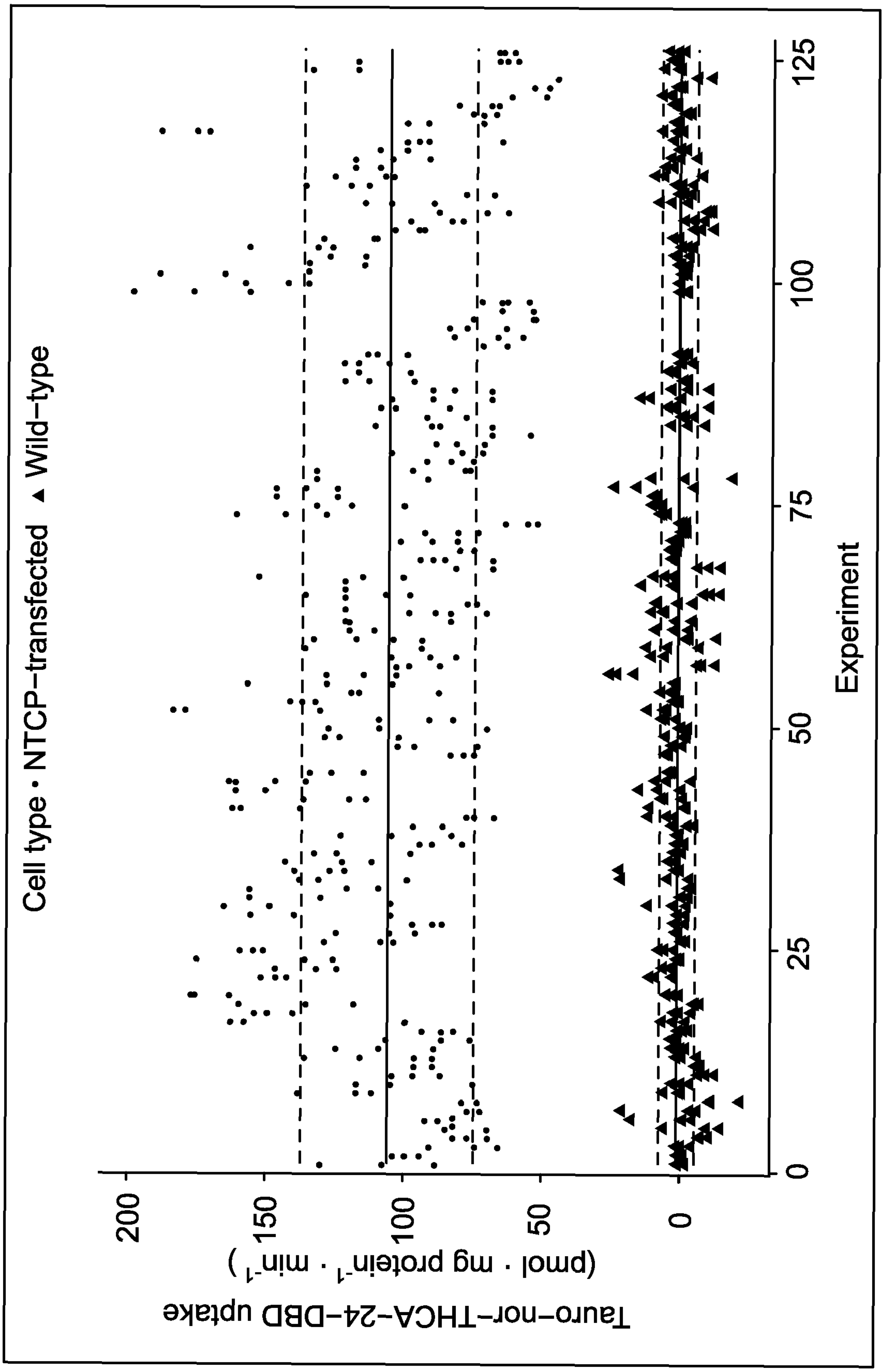
Cell type • NTCP–transfected ▲ Wild–type
Tauro–nor–THCA–24–DBD uptake
(pmol · mg protein-1 · min-1)
200
150
100
50
0
0
25
50
75
100
125
Experiment

FIG. 7

Europäisches Patentamt
European Patent Office
Office européen des brevets

# EUROPEAN SEARCH REPORT

Application Number
EP 21 20 0488

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | M. A. A. VAN DE KLUNDERT ET AL: "Identification of FDA-approved drugs that target hepatitis B virus transcription", JOURNAL OF VIRAL HEPATITIS, vol. 23, no. 3, 12 October 2015 (2015-10-12), pages 191-201, XP055625194, OXFORD, UK ISSN: 1352-0504, DOI: 10.1111/jvh.12479 * page 194; table 1; compounds Manidipine,Cilnidipine * ----- | 1-15 | INV. A61K31/4422 A61P31/20 |
| X | DONKERS JOANNE M. ET AL: "Reduced hepatitis B and D viral entry using clinically applied drugs as novel inhibitors of the bile acid transporter NTCP", SCIENTIFIC REPORTS, vol. 7, no. 1, 1 December 2017 (2017-12-01), XP055850046, DOI: 10.1038/s41598-017-15338-0 Retrieved from the Internet: URL:https://www.nature.com/articles/s41598-017-15338-0.pdf> * page 3; table 1 * ----- | 1,2,5, 10-15 | TECHNICAL FIELDS SEARCHED (IPC) A61K A61P |
| X | WO 2004/024153 A2 (DIAKRON PHARMACEUTICALS INC [US]; BIBBS JEFFREY A [US] ET AL.) 25 March 2004 (2004-03-25) * the whole document * ----- | 14,15 | |

1 The present search report has been drawn up for all claims

| Place of search | Date of completion of the search | Examiner |
|---|---|---|
| The Hague | 14 March 2022 | Megido, Benigno |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 20 0488

This annex lists the patent family members relating to the patent documents cited in the above-mentioned European search report. The members are as contained in the European Patent Office EDP file on
The European Patent Office is in no way liable for these particulars which are merely given for the purpose of information.

14-03-2022

| Patent document cited in search report | Publication date | Patent family member(s) | Publication date |
|---|---|---|---|
| WO 2004024153 A2 | 25-03-2004 | AU 2003270562 A1 | 30-04-2004 |
| | | CA 2498282 A1 | 25-03-2004 |
| | | EP 1542683 A2 | 22-06-2005 |
| | | JP 4897217 B2 | 14-03-2012 |
| | | JP 2006503042 A | 26-01-2006 |
| | | US 8106062 B1 | 31-01-2012 |
| | | WO 2004024153 A2 | 25-03-2004 |

EPO FORM P0459

For more details about this annex : see Official Journal of the European Patent Office, No. 12/82